# EUROPEAN PATENT SPECIFICATION

(11) **EP 1 257 285 B1**
(45) Date of publication and mention of the grant of the patent: **24.01.2007**
(21) Application number: 01905965.8
(22) Date of filing: 21.02.2001
(51) Int. Cl.: A61K 38/17, A61K 39/00, A61K 39/395, C07K 16/18, G01N 33/50, G01N 33/53, A61K 48/00, C12Q 1/68, A61P 35/00

(54) **USE OF BREAST CANCER ASSOCIATED MEMBRANE PROTEINS (BCMP) FOR TREATMENT, PROPHYLAXIS AND DIAGNOSIS OF BREAST CANCER**
VERWENDUNG VON BRUSTKREBS MEMBRANPROTEINE FÜR BEHANDLUNG, PROPHYLAXE UND DIAGNOSE VON BRUSTKREBS
UTILISATION DE PROTEINES MEMBRANAIRES ASSOCIEES AU CANCER DU SEIN (BCMP) POUR LE TRAITEMENT, LA PROPHYLAXIE ET LE DIAGNOSTIC DU CANCER DU SEIN

(30) Priority: 25.02.2000 GB 0004576; 21.12.2000 GB 0031341
(43) Date of publication of application: 20.11.2002
(73) Proprietor: UCB, S.A., 1070 Bruxelles (BE)
(72) Inventor: BOYD, Robert Simon, Oxford GlycoSciences (UK) Ltd, Abingdon, Oxfordshire OX14 4RY (GB); STAMPS, Alasdair Craig, Abingdon Oxfordshire OX14 4RY (GB); TERRETT, Jonathan, Alexander, Abingdon Oxfordshire OX14 4RY (GB)
(74) Representative: Thompson, John
(86) International application number: PCT/GB2001/000748
(87) International publication number: WO 2001/062784

(56) References cited:
- WO-A-98/07749
- WO-A-99/47669
- PAGE MARTIN J ET AL: "Proteomic definition of normal human luminal and myoepithelial breast cells purified from reduction mammoplasties." PROCEEDINGS OF THE NATIONAL ACADEMY OF SCIENCES OF THE UNITED STATES, vol. 96, no. 22, 26 October 1999 (1999-10-26), pages 12589-12594, XP002179217 Oct. 26, 1999 ISSN: 0027-8424 cited in the application

## Description

### INTRODUCTION

The present invention relates to the identification of membrane proteins not previously reported in human breast cancer cells which may form biological targets against which therapeutic antibodies or other pharmaceutical agents can be made, or have utility as diagnostic and prognostic markers for breast cancer and breast cancer metastases.

### BACKGROUND OF THE INVENTION

Breast cancer is the most frequently diagnosed non-skin cancer among women in the United States. It is second only to lung cancer in cancer-related deaths. Approximately 180,000 new cases of breast cancer will be diagnosed in 1997, and about 44,000 women are expected to die from the disease (National Cancer Institute, www.nci.nih.gov, USA, 1999). In the UK, breast cancer is by far the commonest cancer for women, with 34,600 new cases in 1998 (Cancer Research Campaign, www.crc.org.uk, UK, 2000). Ninety-nine percent of breast cancers occur in women. The risk of developing breast cancer steadily increases with age; the lifetime risk of developing breast cancer is estimated to be 1 in 8 for women in the US. The annual cost of breast cancer treatment in the United States is approximately $10 billion (Fuqua, et. al. 2000, American Association for Cancer Research, www.aacr.org, USA). Breast cancer incidence has been rising over the past five decades, but recently it has plateaued. This may reflect a period of earlier detection of breast cancers by mammography. A number of established factors can increase a woman's risk of having the disease. These include older age, history of prior breast cancer, significant radiation exposure, strong family history of breast cancer, upper socioeconomic class, nulliparity, early menarche, late menopause, or age at first pregnancy greater than 30 years. Prolonged use of oral contraceptives earlier in life appears to increase risk slightly. Prolonged postmenopausal oestrogen replacement increases the risk 20 to 40%. It has been speculated that a decrease in the age at menarche, changing birth patterns, or a rise in the use of exogenous oestrogens has contributed to the increase in breast cancer incidence (Fuqua, et. al. 2000, American Association for Cancer Research, www.aacr.org, USA).

### Causes of Breast Cancer

Breast cancer is a heterogeneous disease. Although female hormones play a significant role in driving the origin and evolution of many breast tumours, there are a number of other recognised and unknown factors involved. Perturbations in oncogenes identified include amplification of the HER-2 and the epidermal growth factor receptor genes, and overexpression of cyclin D1. Overexpression of these oncogenes has been associated with a significantly poorer prognosis. Similarly, genetic alterations or the loss of tumour suppressor genes, such as the p53 gene, have been well documented in breast cancer and are also associated with a poorer prognosis. Researchers have identified two genes, called BRCA1 and BRCA2, which are predictive of premenopausal familial breast cancer. Genetic risk assessment is now possible, which may enhance the identification of candidates for chemoprevention trials (Fuqua, et. al. 2000, American Association for Cancer Research, www.aacr.org, USA).

### Diagnosis

Early diagnosis of breast cancer is vital to secure the most favourable outcome for treatment. Many countries with advanced healthcare systems have instituted screening programmes for breast cancer. This typically takes the form of regular x-ray of the breast (mammography) during the 50-60 year old age interval where greatest benefit for this intervention has been shown. Some authorities have advocated the extension of such programmes beyond 60 and to the 40-49 age group. Health authorities in many countries have also promoted the importance of regular breast self-examination by women. Abnormalities detected during these screeening procedures and cases presenting as symptomatic would normally be confirmed by aspiration cytology, core needle biopsy with a stereotactic or ultrasound technique for nonpalpable lesions, or incisional or excisional biopsy. At the same time other information relevant to treatment options and prognosis, such as oestrogen (ER) and progesterone receptor (PR) status would be determined (National Cancer Institute, USA, 2000, Breast Cancer PDQ, www.nci.nih.gov).

### Disease Staging and Prognosis

Staging is the process of finding out how far the cancer has spread. The staging system of the American Joint Committee on Cancer (AJCC), also known as the TNM system, is the one used most often for breast cancer. The TNM system for staging gives three key pieces of information:

The letter T followed by a number from 0 to 4 describes the tumour's size and spread to the skin or chest wall under the breast. A higher number means a larger tumour and/or more spread to tissues near the breast.

The letter N, followed by a number from 0 to 3, indicates whether the cancer has spread to lymph nodes near the breast and, if so, whether the affected nodes are adhered to other structures under the arm.

The letter M, followed by a 0 or 1, shows whether the cancer has metastasized to other organs of the body or to lymph nodes that are not next to the breast.

To make this information somewhat clearer, the TNM descriptions can be grouped together into a simpler set of stages, labeled stage 0 through stage IV (0-4). In general, the lower the number, the less the cancer has spread. A higher number, such as stage IV (4), means a more serious cancer. (American Cancer Society, 2000, USA, www.cancer.org)

### Breast Cancer Survival by Stage

| **Stage** | **5-year relative survival rate** |
|---|---|
| 0 | 100% |
| I | 98% |
| IIA | 88% |
| IIB | 76% |
| IIIA | 56% |
| IIIB | 49% |
| IV | 16% |

| | |
|---|---|
| (American Cancer Society, 2000, USA, www.cancer.org) | |

Although anatomic stage (size of primary tumour, axillary lymph node involvement) is an important prognostic factor, other characteristics may have predictive value. For example studies from the National Surgical Adjuvant Breast and Bowel Project (NSABP) and the International Breast Cancer Study Group (IBCSG) have shown that tumour nuclear grade and histologic grade, respectively, are important indicators of outcome following adjuvant therapy for breast cancer. There is substantial evidence that oestrogen receptor status and measures of proliferative capacity of the primary tumour (thymidine labeling index or flow cytometric measurements of S-phase and ploidy) may have important independent predictive value. In stage II disease, the PR status may have greater prognostic value than the ER status. Tumour vascularisation, c-erbB-2, c-myc, p53 expression, and lymphatic vessel invasion may also be prognostic indicators in patients with breast cancer (National Cancer Institute, USA, 2000, Breast Cancer PDQ, www.nci.org and references therein).

### Treatment

Surgery, radiation therapy, hormone therapy, and chemotherapy are the most common treatments for breast cancer.

Surgery Most women with breast cancer will have some type of surgery. The purpose of surgery is to remove as much of the cancer as possible. This may be in the form of lumpectomy or more radical mastectomy with breast reconstruction. Surgery may also be combined with other treatments like chemotherapy, hormone therapy, or radiation therapy. Surgery may also be done to find out whether breast cancer has spread to the lymph nodes under the arm (axillary dissection), to restore a more normal appearance (reconstructive surgery), or to relieve symptoms of advanced cancer.

Chemotherapy Chemotherapy is the use of anticancer drugs to kill cancer cells. When chemotherapy is given after surgery (adjuvant therapy) it can reduce the chance of cancer recurrence. Chemotherapy can also be used as the main treatment for women whose cancer is widespread when it is found, or spreads widely after initial treatment. Neoadjuvant chemotherapy is given before surgery, often to shrink the tumour and make it easier to remove. Chemotherapy is given in cycles, with each period of treatment followed by a recovery period. The total course lasts three to six months. It is often more effective to use several drugs, rather than a single drug alone. The most commonly used combinations are: cyclophosphamide, methotrexate, and fluorouracil (CMF) cyclophosphamide, doxorubicin (Adriamycin), and fluorouracil (CAF) doxorubicin (Adriamycin) and cyclophosphamide (AC), with or without paclitaxel (Taxol) doxorubicin (Adriamycin), followed by CMF.

Radiation therapy Radiation therapy is commonly applied in breast cancer treatment. It may be used to reduce the size of a tumour before surgery or to destroy cancer cells remaining in the breast, chest wall, or underarm area after surgery.

Hormone therapy and Chemoprevention The hormone oestrogen can increase the growth of breast cancer cells in some women. A drug such as tamoxifen, which blocks the effect of oestrogen, is given to counter this growth. Another newer drug, raloxifene, also blocks the effect of oestrogen on breast tissue and breast cancer. There is increasing evidence that these anti-oestrogen treatments may also have a role in chemoprevention of breast cancer in high-risk individuals.

Immunotherapy Trastuzumab (Herceptin) is a new immunotherapeutic agent that attaches to a growth factor receptor known as c-erbB2/HER2/neu, which is present in small amounts on the surface of normal breast cells and at much higher levels in some breast cancers. This protein can cause the cancer to grow and spread faster. Herceptin can stop the c-erbB2/HER2/neu protein from promoting breast cancer cell growth. It may also help the immune system to better attack the cancer. Herceptin is currently started after standard hormonal or chemotherapy is no longer working (American Cancer Society, 2000, USA, www.cancer.org).

### Therapeutic Challenges

The major challenges in breast cancer treatment are to improve early detection rates, to find new non-invasive markers that can be used to follow disease progression and identify relapse, and to find improved and less toxic therapies, especially for more advanced disease where 5 year survival is still very poor. There is a great need to identify targets which are more specific to the cancer cells, ideally ones which are expressed on the surface of the tumour cells so that they can be attacked by promising new approaches like immunotherapeutics and targeted toxins.

### SUMMARY OF THE INVENTION

The present invention provides methods and for uses for screening, diagnosis, prognosis and therapy of breast cancer, for monitoring the effectiveness of breast cancer treatment, and for drug development for treatment of breast cancer.

We have used mass spectrometry to identify peptides generated by gel electrophoresis and tryptic digest of membrane protein extracts of laboratory cultured human mammary cell lines. Peptide sequences were compared to existing cDNA databases and corresponding gene sequences identified. Many of these have not been previously reported in breast cell membranes and represent a new set of proteins of potential diagnostic and/or therapeutic value.

Thus, a first aspect of the invention provides methods for diagnosis of breast cancer that comprises analysing a sample of breast tissue by one-dimensional electrophoresis to detect a Breast Cancer-associated Membrane Protein (BCMP). Only BCMP 17 (BST-2) is intended to form part of the invention disclosed herein. The term a "BCMP" or "BCMPs" is only intended to refer to BCMP 17 (BST-2). These methods are also suitable for screening, prognosis, monitoring the results of therapy, drug development and discovery of new targets for drug treatment.

A second aspect of the invention provides the use of compounds as defined in claims 1, 4 and 19 for the manufacture of a medicament useful in methods of treating breast cancer, comprising administering to a patient a therapeutically effective amount of a compound that modulates (e.g., upregulates or downregulates) or complements the expression or the biological activity (or both) of a BCMP in patients having breast cancer, in order to (a) prevent the onset or development of breast cancer; (b) prevent the progression of breast cancer; or (c) ameliorate the symptoms of breast cancer.

A third aspect of the invention provides methods of screening for compounds that modulate (e.g., upregulate or downregulate) the expression or biological activity of a BCMP.

A fourth aspect of the invention provides the use of monoclonal and polyclonal antibodies capable of immunospecific binding to a BCMP.

Thus, in a fifth aspect, the present invention provides a method for screening for and/or diagnosis of breast cancer in a human subject, which method comprises the step of identifying the presence or absence of BCMP 17 as defined by an apparent molecular weight of 15kDa, a tryptic digest peptide sequence of LQDASAEVER and SwissProt Accession Number Q10589 in a biological sample obtained from said human subject.

In a sixth aspect, the present invention provides a method for monitoring and/or assessing breast cancer treatment in a human subject, which comprises the step of identifying the presence or absence of the BCMPs as defined above in a biological sample obtained from said human subject.

In a seventh aspect, the present invention provides a method for identifying the presence or absence of metastatic breast cancer cells in a biological sample obtained from a human subject, which comprises the step of identifying the presence or absence of the BCMPs as defined above.

In an eighth aspect, the present invention provides a method for monitoring and/or assessing breast cancer treatment in a human subject, which comprises the step of determining whether the BCMPs as defined aboveis increased/decreased in a biological sample obtained from a patient.

The biological sample used can be from any source such as a serum sample or a tissue sample, e.g. breast tissue. For instance, when looking for evidence of metastatic breast cancer, one would look at major sites of breast metastasis, e.g. lymph nodes, liver, lung and/or bone.

These aspects of the present invention are set out in the claims and explained below.

### BRIEF DESCRIPTION OF THE FIGURES

Figures 1 to 8 and Figure 10 are not intended to form part of the invention disclosed herein.

Figure 9 shows the level of mRNA expression in various tissues for BCMP 17.

### DETAILED DESCRIPTION OF THE INVENTION

The invention described in detail below provides methods for clinical screening, diagnosis and prognosis of breast cancer in a mammalian subject for identifying patients most likely to respond to a particular therapeutic treatment, for monitoring the results of breast cancer therapy, for drug screening and drug development. The invention also encompasses the use of therapeutic compositions to a mammalian subject to treat or prevent breast cancer. The mammalian subject may be a non-human mammal, but is preferably human, more preferably a human adult, i.e. a human subject at least 21 (more preferably at least 35, at least 50, at least 60, at least 70, or at least 80) years old. For clarity of disclosure, and not by way of limitation, the invention will be described with respect to the analysis of breast tissue samples. However, as one skilled in the art will appreciate, the assays and techniques described below can be applied to other types of patient samples, including a body fluid (*e.g*. blood, serum, plasma or saliva), a tissue sample from a patient at risk of having breast cancer (*e.g.* a biopsy such as a breast tissue biopsy) or homogenate thereof. The methods of the present invention are specially suited for screening, diagnosis and prognosis of a living subject, but may also be used for postmortem diagnosis in a subject, for example, to identify family members at risk of developing the same disease.

As used herein, breast tissue refers to the breast itself, as well as the tissue adjacent to and/or within the strata underlying the breast.

### Breast Cancer-associated Membrane Proteins (BCMPs)

In one aspect of the invention, one-dimensional electrophoresis is used to analyse breast tissue from a subject, preferably a living subject, in order to measure the expression of one or more Breast Cancer-associated Membrane Proteins (BCMPs) for screening or diagnosis of breast cancer, to determine the prognosis of a breast cancer patient, to monitor the effectiveness of breast cancer therapy, or for drug development. As used herein, "one-dimensional electrophoresis" (1D-electrophoresis) means a technique comprising denaturing electrophoresis; this generates a one-dimensional gel (1D-gel) containing a plurality of separated proteins. Preferably, the step of denaturing electrophoresis uses polyacrylamide electrophoresis in the presence of sodium dodecyl sulfate (SDS-PAGE). Especially preferred are the highly accurate and automatable methods and apparatus ("the Preferred Technology") described in WO 98/23950, with particular reference to the preferred protocol at pages 19-29. Briefly, the Preferred Technology provides efficient, computer-assisted methods and apparatus for identifying, selecting and characterising biomolecules in a biological sample. A one-dimensional array is generated by separating biomolecules on a one-dimensional gel according to their electrophoretic mobility. A computer-generated digital profile of the array is generated, representing the identity, apparent molecular weight of a plurality of biomolecules detected in the one-dimensional array, thereby permitting computer-mediated comparison of profiles from multiple biological samples, as well as computer aided excision of separated proteins of interest.

A preferred scanner for detecting fluorescently labelled proteins is described in WO 96/36882 and in the Ph.D. thesis of David A. Basiji, entitled "Development of a High-throughput Fluorescence Scanner Employing Internal Reflection Optics and Phase-sensitive Detection (Total Internal Reflection, Electrophoresis)", University of Washington (1997), Volume 58/12-B of Dissertation Abstracts International, page 6686. These documents describe an image scanner designed specifically for automated, integrated operation at high speeds. The scanner can image gels that have been stained with fluorescent dyes or silver stains, as well as storage phosphor screens. The Basiji thesis provides a phase-sensitive detection system for discriminating modulated fluorescence from baseline noise due to laser scatter or homogeneous fluorescence, but the scanner can also be operated in a non-phase-sensitive mode: This phase-sensitive detection capability would increase the sensitivity of the instrument by an order of magnitude or more compared to conventional fluorescence imaging systems. The increased sensitivity would reduce the sample-preparation load on the upstream instruments while the enhanced image quality simplifies image analysis downstream in the process.

A more highly preferred scanner is the Apollo 2 scanner (Oxford Glycosciences, Oxford, UK), which is a modified version of the above described scanner. In the Apollo 2 scanner, the gel is transported through the scanner on a precision lead-screw drive system. This is preferable to laying the glass plate on the belt-driven system that is described in the Basiji thesis as it provides a reproducible means of accurately transporting the gel past the imaging optics.

In the Apollo 2 scanner, the gel is secured against three alignment stops that rigidly hold the glass plate in a known position. By doing this in conjunction with the above precision transport system, the absolute position of the gel can be predicted and recorded. This ensures that co-ordinates of each feature on the gel can be determined more accurately and communicated, if desired, to a cutting robot for excision of the feature. In the Apollo 2 scanner, the carrier that holds the gel has four integral fluorescent markers for use to correct the image geometry. These markers are a quality control feature that confirms that the scanning has been performed correctly.

In comparison to the scanner described in the Basiji thesis, the optical components of the Apollo 2 scanner have been inverted. In the Apollo 2 scanner, the laser, mirror, waveguide and other optical components are above the glass plate being scanned. The scanner described in the Basiji thesis has these components underneath. In the Apollo 2 scanner, the glass plate is mounted onto the scanner gel side down, so that the optical path remains through the glass plate. By doing this, any particles of gel that may break away from the glass plate will fall onto the base of the instrument rather than into the optics. This does not affect the functionality of the system, but increases its reliability.

Still more preferred is the Apollo3 scanner, in which the signal output is digitized to the full 16-bit data without any peak saturation or without square root encoding of the signal. A compensation algorithm has also been applied to correct for any variation in detection sensitivity along the path of the scanning beam. This variation is due to anomalies in the optics and differences in collection efficiency across the waveguide. A calibration is performed using a perspex plate with an even fluorescence throughout. The data received from a scan of this plate are used to determine the multiplication factors needed to increase the signal from each pixel level to a target level. These factors are then used in subsequent scans of gels to remove any internal optical variations.

As used herein, the term "Breast Cancer-associated Membrane Protein" (BCMP) refers to a feature *(e.g.,* a band in a 1D gel), detectable by 1D electrophoresis of a breast tissue sample, that is present in breast tissue from a subject having breast cancer.

The BCMPs disclosed herein have been identified in membrane protein extracts of laboratory cultured human mammary cell lines through the methods and apparatus of the Preferred Technology (generally 1D gel electrophoresis and tryptic digest of membrane protein extracts of laboratory cultured human mammary cell lines). Peptide sequences were compared to the SWISS-PROT and trEMBL databases (held by the Swiss Institute of Bioinformatics (SIB) and the European Bioinformatics Institue (EBI) which are available at http://www.expasy.com/) and the GenBank database (held by the National Institute of Health (NIH) which is available at http://www.ncbi.nlm.nih.gov/GenBank/) and corresponding genes identified. Published reports and databases, including databases of proteins expressed in normal human breast luminal epithelial cells (Page *et al. Proc Natl Acad Sci U S A.* 1999 **96**(22):12589-94; GB patent application no. 9919258.5) were searched to establish whether the products of any of the identified genes had been previously demonstrated to be expressed in the membrane of human breast cells or human breast cancer cells. Two groups of BCMPs have been identified: (1) protein sequences matching conceptual translations of cDNAs for which no protein or biological function has been described, for which the present invention defines the existence of the protein product and its localisation in the membranes of human breast cancer cells; (2) known proteins which have not previously been described in breast cell membranes, which the present invention shows may be additionally involved in human breast cancer. BCMP 17 finds utility as markers for breast cells, especially breast cancer cells. The amino acid sequence of those BCMP 17 is identified by a Swiss Prot Accession Number Q10589 and the sequence is incorporated herein by reference. The apparent molecular weight is 15kDa and the amino acid sequences of tryptic digest peptides of BCMP 17 identified by tandem mass spectrometry and database searching as described in the Examples, *infra,* is LQDASAEVER.

BCMP 17 described above can be used in the diagnosis and therapy of breast cancer mentioned in more detail below.

For any given BCMP, the detected level obtained upon analyzing breast tissue from subjects having breast cancer relative to the detected level obtained upon analyzing breast tissue from subjects free from breast cancer will depend upon the particular analytical protocol and detection technique that is used, provided that such BCMP is differentially expressed between normal and disease tissue. Accordingly, the present invention contemplates that each laboratory will establish a reference range for each BCMP in subjects free from breast cancer according to the analytical protocol and detection technique in use, as is conventional in the diagnostic art. Preferably, at least one control positive breast tissue sample from a subject known to have breast cancer or at least one control negative breast tissue sample from a subject known to be free from breast cancer (and more preferably both positive and negative control samples) are included in each batch of test samples analysed.

In one embodiment, the level of expression of a protein is determined relative to a background value, which is defined as the level of signal obtained from a proximal region of the image that (a) is equivalent in area to the particular feature in question; and (b) contains no discernable protein feature.

BCMPs can be used for detection, prognosis, diagnosis, or monitoring of breast cancer or for drug development. In one embodiment of the invention, breast tissue from a subject (*e.g.*, a subject suspected of having breast cancer) is analysed by 1D electrophoresis for detection of one or more of the BCMPs. A decreased abundance of said one or more BCMPs in the breast tissue from the subject relative to breast tissue from a subject or subjects free from breast cancer (*e.g*., a control sample) or a previously determined reference range indicates the presence or absence of breast cancer.

As will be evident to one of skill in the art, a given BCMP can be described according to the data provided for that BCMP. The BCMP is a protein comprising a peptide sequence described for that BCMP (preferably comprising a plurality of, more preferably all of, the peptide sequences described for that BCMP).

In one embodiment, breast tissue from a subject is analysed for quantitative detection of the BCMPs, wherein a change in abundance of the BCMP in the breast tissue from the subject relative to breast tissue from a subject or subjects free from breast cancer (*e.g.*, a control sample or a previously determined reference range) indicates the presence of breast cancer.

For each BCMP the present invention additionally provides the use of: (a) a preparation comprising the isolated BCMP; (b) a preparation comprising one or more fragments of the BCMP; and (c) antibodies that bind to said BCMP, to said fragments, or both to said BCMP and to said fragments. As used herein, a BCMP is "isolated" when it is present in a preparation that is substantially free of contaminating proteins, *i.e.*, a preparation in which less than 10% (preferably less than 5%, more preferably less than 1%) of the total protein present is contaminating protein(s). A contaminating protein is a protein having a significantly different amino acid sequence from that of the isolated BCMP, as determined by mass spectral analysis. As used herein, a "significantly different" sequence is one that permits the contaminating protein to be resolved from the BCMP by mass spectral analysis, performed according to the Reference Protocol.

The BCMPs can be assayed by any method known to those skilled in the art, including but not limited to, the Preferred Technology described herein, kinase assays, enzyme assays, binding assays and other functional assays, immunoassays, and western blotting. In one embodiment, the BCMPs are separated on a 1-D gel by virtue of their MWs and visualized by staining the gel. In one embodiment, the BCMPs are stained with a fluorescent dye and imaged with a fluorescence scanner: Sypro Red (Molecular Probes, Inc., Eugene, Oregon) is a suitable dye for this purpose. A preferred fluorescent dye is disclosed in U.S. Application No. 09/412,168, filed on October 5, 1999, which is incorporated herein by reference in its entirety.

Alternatively, BCMPs can be detected in an immunoassay. In one embodiment, an immunoassay is performed by contacting a sample from a subject to be tested with an anti-BCMP antibody under conditions such that immunospecific binding can occur if the BCMP is present, and detecting or measuring the amount of any immunospecific binding by the antibody. Anti-BCMP antibodies can be produced by the methods and techniques taught herein.

In one embodiment, binding of antibody in tissue sections can be used to detect aberrant BCMP localization or an aberrant level of one or more BCMPs. In a specific embodiment, antibody to a BCMP can be used to assay a patient tissue (*e.g.*, a breast tissue biopsy) for the level of the BCMP where an aberrant level of BCMP is indicative of breast cancer. As used herein, an "aberrant level" means a level that is increased or decreased compared with the level in a subject free from breast cancer or a reference level. If desired, the comparison can be performed with a matched sample from the same subject, taken from a portion of the body not affected by breast cancer.

Any suitable immunoassay can be used, including, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, a BCMP can be detected in a fluid sample (e.g., blood, urine, or breast tissue homogenate) by means of a two-step sandwich assay. In the first step, a capture reagent (e.g., an anti-BCMP antibody) is used to capture the BCMP. The capture reagent can optionally be immobilized on a solid phase. In the second step, a directly or indirectly labeled detection reagent is used to detect the captured BCMP. In one embodiment, the detection reagent is a lectin. Any lectin can be used for this purpose that preferentially binds to the BCMP rather than to other isoforms that have the same core protein as the BCMP or to other proteins that share the antigenic determinant recognized by the antibody. In a preferred embodiment, the chosen lectin binds to the BCMP with at least 2-fold greater affinity, more preferably at least 5-fold greater affinity, still more preferably at least 10-fold greater affinity, than to said other isoforms that have the same core protein as the BCMP or to said other proteins that share the antigenic determinant recognized by the antibody. Based on the present description, a lectin that is suitable for detecting a given BCMP can readily be identified by methods well known in the art, for instance upon testing one or more lectins enumerated in Table I on pages 158-159 of Sumar et al., Lectins as Indicators of Disease-Associated Glycoforms, *In*: Gabius H-J & Gabius S (eds.), 1993, Lectins and Glycobiology, at pp. 158-174. In an alternative embodiment, the detection reagent is an antibody, e.g., an antibody that immunospecifically detects other post-translational modifications, such as an antibody that immunospecifically binds to phosphorylated amino acids. Examples of such antibodies include those that bind to phosphotyrosine (BD Transduction Laboratories, catalog nos.: P1 1230-050/P11230-150; P11120; P38820; P39020), those that bind to phosphoserine (Zymed Laboratories Inc., South San Francisco, CA, catalog no. 61-8100) and those that bind to phosphothreonine (Zymed Laboratories Inc., South San Francisco, CA, catalogue nos. 71-8200, 13-9200).

If desired, a gene encoding a BCMP, a related gene, or related nucleic acid sequences or subsequences, including complementary sequences, can also be used in hybridization assays. A nucleotide encoding a BCMP, or subsequences thereof comprising at least 8 nucleotides, preferably at least 12 nucleotides, and most preferably at least 15 nucleotides can be used as a hybridization probe. Hybridization assays can be used for detection, prognosis, diagnosis, or monitoring of conditions, disorders, or disease states, associated with aberrant expression of genes encoding BCMPs, or for differential diagnosis of subjects with signs or symptoms suggestive of breast cancer. In particular, such a hybridization assay can be carried out by a method comprising contacting a subject's sample containing nucleic acid with a nucleic acid probe capable of hybridizing to a DNA or RNA that encodes a BCMP, under conditions such that hybridization can occur, and detecting or measuring any resulting hybridization. Nucleotides can be used for therapy of subjects having breast cancer, as described below.

The invention also provides the use of diagnostic kits, comprising an anti-BCMP antibody. In addition, such a kit may optionally comprise one or more of the following: (1) instructions for using the anti-BCMP antibody for diagnosis, prognosis, therapeutic monitoring or any combination of these applications; (2) a labeled binding partner to the antibody; (3) a solid phase (such as a reagent strip) upon which the anti-BCMP antibody is immobilized; and (4) a label or insert indicating regulatory approval for diagnostic, prognostic or therapeutic use or any combination thereof. If no labeled binding partner to the antibody is provided, the anti-BCMP antibody itself can be labeled with a detectable marker, *e.g.,* a chemiluminescent, enzymatic, fluorescent, or radioactive moiety.

The invention also provides the use of a kit comprising a nucleic acid probe capable of hybridizing to RNA encoding a BCMP. In a specific embodiment, a kit comprises in one or more containers a pair of primers (*e.g.*, each in the size range of 6-30 nucleotides, more preferably 10-30 nucleotides and still more preferably 10-20 nucleotides) that under appropriate reaction conditions can prime amplification of at least a portion of a nucleic acid encoding a BCMP, such as by polymerase chain reaction (see, *e.g.,* Innis et al., 1990, PCR Protocols, Academic Press, Inc., San Diego, CA), ligase chain reaction (see EP 320,308) use of Qβ replicase, cyclic probe reaction, or other methods known in the art.
A kit can optionally further comprise a predetermined amount of an isolated BCMP protein or a nucleic acid encoding a BCMP, *e.g.,* for use as a standard or control.

### Use in Clinical Studies

The diagnostic methods of the present invention can assist in monitoring a clinical study, *e.g.* to evaluate drugs for therapy of breast cancer. In one embodiment, candidate molecules are tested for their ability to restore BCMP levels in a subject having breast cancer to levels found in subjects free from breast cancer or, in a treated subject (*e.g.* after treatment with taxol or doxorubacin), to preserve BCMP levels at or near non-breast cancer values. The levels ofBCMPs can be assayed.

In another embodiment, the methods of the present invention are used to screen candidates for a clinical study to identify individuals having breast cancer; such individuals can then be excluded from the study or can be placed in a separate cohort for treatment or analysis. If desired, the candidates can concurrently be screened to identify individuals with breast cancer; procedures for these screens are well known in the art.

### Purification of BCMPs

In particular aspects, the invention provides the use of isolated BCMPs, preferably human BCMPs, and fragments and derivatives thereof which comprise an antigenic determinant (*i.e*., can be recognised by an antibody) or which are otherwise functionally active, as well as nucleic acid sequences encoding the foregoing. "Functionally active" as used herein refers to material displaying one or more functional activities associated with a full-length (wild-type) BCMP, *e.g.*, binding to a BCMP substrate or BCMP binding partner, antigenicity (binding to an anti-target antibody), immunogenicity, enzymatic activity etc.

In specific embodiments, the invention provides the use of peptide fragments of a BCMP comprising at least 5 amino acids, at least 10 amino acids, at least 50 amino acids, or at least 75 amino acids. Fragments lacking some or all of the regions of a BCMP are also provided, as are proteins (*e.g.,* fusion proteins) comprising such fragments. Nucleic acids encoding the foregoing are provided.

Once a recombinant nucleic acid which encodes the BCMP, a portion of the BCMP, or a precursor of the BCMP is identified, the gene product can be analysed. This is achieved by assays based on the physical or functional properties of the product, including radioactive labeling of the product followed by analysis by gel electrophoresis, immunoassay, etc.

The BCMPs identified herein can be isolated and purified by standard methods including chromatography (*e.g.,* ion exchange, affinity, and sizing column chromatography), centrifugation, differential solubility, or by any other standard technique for the purification of proteins.

Alternatively, once a recombinant nucleic acid that encodes the BCMP is identified, the entire amino acid sequence of the BCMP can be deduced from the nucleotide sequence of the gene-coding region contained in the recombinant nucleic acid. As a result, the protein can be synthesized by standard chemical methods known in the art (*e.g.,* see Hunkapiller et al., 1984, Nature 310:105-111).

In another alternative embodiment, native BCMPs can be purified from natural sources, by standard methods such as those described above (e.g., immunoaffinity purification).

The invention thus provides the use of an isolated BCMP, an isolated BCMP-related polypeptide, and an isolated derivative or fragment of a BCMP or a BCMP-related polypeptide; any of the foregoing can be produced by recombinant DNA techniques or by chemical synthetic methods.

### Isolation Of DNA Encoding a BCMP

Specific embodiments for the cloning of a gene encoding a BCMP, are presented below by way of example and not of limitation.

Nucleotide sequences, including DNA and RNA, and comprising a sequence encoding a BCMP or a fragment thereof, or a BCMP-related polypeptide, may be synthesized using methods known in the art, such as using conventional chemical approaches or polymerase chain reaction (PCR) amplification. The nucleotide sequences also permit the identification and cloning of the gene encoding a BCMP homolog or BCMP ortholog including, for example, by screening cDNA libraries, genomic libraries or expression libraries.

For example, to clone a gene encoding a BCMP by PCR techniques, anchored degenerate oligonucleotides (or a set of most likely oligonucleotides) can be designed for all BCMP peptide fragments identified as part of the same protein. PCR reactions under a variety of conditions can be performed with relevant cDNA and genomic DNAs (e.g., from breast tissue or from cells of the immune system) from one or more species. Also vectorette reactions can be performed on any available cDNA and genomic DNA using the oligonucleotides (which preferably are nested) as above. Vectorette PCR is a method that enables the amplification of specific DNA fragments in situations where the sequence of only one primer is known. Thus, it extends the application of PCR to stretches of DNA where the sequence information is only available at one end. (Arnold C, 1991, PCR Methods Appl. 1(1):39-42; Dyer KD, Biotechniques, 1995, 19(4):550-2). Vectorette PCR may be performed with probes that are, for example, anchored degenerate oligonucleotides (or most likely oligonucleotides) coding for BCMP peptide fragments, using as a template a genomic library or cDNA library pools.

Anchored degenerate oligonucleotides (and most likely oligonucleotides) can be designed for all BCMP peptide fragments. These oligonucleotides may be labeled and hybridized to filters containing cDNA and genomic DNA libraries. Oligonucleotides to different peptides from the same protein will often identify the same members of the library. The cDNA and genomic DNA libraries may be obtained from any suitable or desired mammalian species, for example from humans.

Nucleotide sequences comprising a nucleotide sequence encoding a BCMP or BCMP fragment of the present invention are useful for their ability to hybridize selectively with complementary stretches of genes encoding other proteins. Depending on the application, a variety of hybridization conditions may be employed to obtain nucleotide sequences at least 30%, 35%, 40%, 45%, 50%, 55%, 60%, 65%, 70%, 75%, 80%, 85%, 90%, 95%, or 99% identical, or 100% identical, to the sequence of a nucleotide encoding a BCMP.

For a high degree of selectivity, relatively stringent conditions are used to form the duplexes, such as low salt or high temperature conditions. As used herein, "highly stringent conditions" means hybridization to filter-bound DNA in 0.5 M NaHPO₄, 7% sodium dodecyl sulfate (SDS), 1 mM EDTA at 65 °C, and washing in 0.1xSSC/0.1% SDS at 68 °C (Ausubel F.M. et al., eds., 1989, Current Protocols in Molecular Biology, Vol. I, Green Publishing Associates, Inc., and John Wiley & Sons, Inc., New York, at p. 2.10.3; incorporated herein by reference in its entirety.) For some applications, less stringent conditions for duplex formation are required. As used herein "moderately stringent conditions" means washing in 0.2xSSC/0.1% SDS at 42 °C (Ausubel et al., *1989, supra).* Hybridization conditions can also be rendered more stringent by the addition of increasing amounts of formamide, to destabilize the hybrid duplex. Thus, particular hybridization conditions can be readily manipulated, and will generally be chosen depending on the desired results. In general, convenient hybridization temperatures in the presence of 50% formamide are: 42 °C for a probe which is 95 to 100% identical to the fragment of a gene encoding a BCMP, 37°C for 90 to 95% identity and 32 °C for 70 to 90% identity.

In the preparation of genomic libraries, DNA fragments are generated, some of which will encode parts or the whole of a BCMP. Any suitable method for preparing DNA fragments may be used in the present invention. For example, the DNA may be cleaved at specific sites using various restriction enzymes. Alternatively, one may use DNAse in the presence of manganese to fragment the DNA, or the DNA can be physically sheared, as for example, by sonication. The DNA fragments can then be separated according to size by standard techniques, including but not limited to agarose and polyacrylamide gel electrophoresis, column chromatography and sucrose gradient centrifugation. The DNA fragments can then be inserted into suitable vectors, including but not limited to plasmids, cosmids, bacteriophages lambda or T₄, and yeast artificial chromosome (YAC). (See, e.g., Sambrook et al., 1989, Molecular Cloning, A Laboratory Manual, 2^{nd} Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II; Ausubel F.M. et al., eds., 1989, Current Protocols in Molecular Biology, Vol. I, Green Publishing Associates, Inc., and John Wiley & sons, Inc., New York). The genomic library may be screened by nucleic acid hybridization to labeled probe (Benton and Davis, 1977, Science 196:180; Grunstein and Hogness, 1975, Proc. Natl. Acad. Sci. U.S.A. 72:3961).

Based on the present description, the genomic libraries may be screened with labeled degenerate oligonucleotide probes corresponding to the amino acid sequence of any peptide of the BCMP using optimal approaches well known in the art. Any probe used is at least 10 nucleotides, at least 15 nucleotides, at least 20 nucleotides, at least 25 nucleotides, at least 30 nucleotides, at least 40 nucleotides, at least 50 nucleotides, at least 60 nucleotides, at least 70 nucleotides, at least 80 nucleotides, or at least 100 nucleotides. Preferably a probe is 10 nucleotides or longer, and more preferably 15 nucleotides or longer.

BCMP 17 disclosed herein was found to correspond to isoforms of previously identified proteins encoded by genes whose sequences are publicly known. (Sequence analysis and protein identification of BCMPs was carried out using the methods described in Examples). To screen such a gene, any probe may be used that is complementary to the gene or its complement; preferably the probe is 10 nucleotides or longer, more preferably 15 nucleotides or longer. The SWISS-PROT and trEMBL databases (held by the Swiss Institute of Bioinformatics (SIB) and the European Bioinformatics Institute (EBI) -- which are available at http://www.expasy.ch/) and the GenBank database (held by the National Institute of Health (NIH) which is available at http://www.ncbi.nlm.nih.gov/) provide protein sequences for the BCMPs.

When a library is screened, clones with inserted DNA encoding the BCMP or a fragment thereof will hybridize to one or more members of the corresponding set of degenerate oligonucleotide probes (or their complement). Hybridization of such oligonucleotide probes to genomic libraries is carried out using methods known in the art. For example, hybridization with one of the above-mentioned degenerate sets of oligonucleotide probes, or their complement (or with any member of such a set, or its complement) can be performed under highly stringent or moderately stringent conditions as defined above, or can be carried out in 2X SSC, 1.0% SDS at 50°C and washed using the washing conditions described supra for highly stringent or moderately stringent hybridization.

In yet another aspect, clones containing nucleotide sequences encoding the entire BCMP, a fragment of a BCMP, a BCMP-related polypeptide, or a fragment of a BCMP-related polypeptide any of the foregoing may also be obtained by screening expression libraries. For example, DNA from the relevant source is isolated and random fragments are prepared and ligated into an expression vector (*e.g*., a bacteriophage, plasmid, phagemid or cosmid) such that the inserted sequence in the vector is capable of being expressed by the host cell into which the vector is then introduced. Various screening assays can then be used to select for the expressed BCMP or BCMP-related polypeptides. In one embodiment, the various anti-BCMP antibodies can be used to identify the desired clones using methods known in the art. See, for example, Harlow and Lane, 1988, Antibodies: A Laboratory Manual, Cold Spring Harbor Laboratory Press, Cold Spring Harbor, NY, Appendix IV. Colonies or plaques from the library are brought into contact with the antibodies to identify those clones that bind antibody.

In an embodiment, colonies or plaques containing DNA that encodes a BCMP, a fragment of a BCMP, a BCMP-related polypeptide, or a fragment of a BCMP-related polypeptide can be detected using DYNA Beads according to Olsvick et al., 29^{th} ICAAC, Houston, Tex. 1989, incorporated herein by reference. Anti-BCMP antibodies are crosslinked to tosylated DYNA Beads M280, and these antibody-containing beads are then contacted with colonies or plaques expressing recombinant polypeptides. Colonies or plaques expressing a BCMP or BCMP-derived polypeptide are identified as any of those that bind the beads.

Alternatively, the anti-BCMP antibodies can be nonspecifically immobilized to a suitable support, such as silica or Celite® resin. This material is then used to adsorb to bacterial colonies expressing the BCMP protein or BCMP-related polypeptide as described herein.

In another aspect, PCR amplification may be used to isolate from genomic DNA a substantially pure DNA *(i.e.,* a DNA substantially free of contaminating nucleic acids) encoding the entire BCMP or a part thereof Preferably such a DNA is at least 95% pure, more preferably at least 99% pure. Oligonucleotide sequences, degenerate or otherwise, that correspond to peptide sequences of BCMPs disclosed herein can be used as primers.

PCR can be carried out, e.g., by use of a Perkin-Ehner Cetus thermal cycler and Taq polymerase (Gene Amp® or AmpliTaq DNA polymerase). One can choose to synthesize several different degenerate primers, for use in the PCR reactions. It is also possible to vary the stringency of hybridization conditions used in priming the PCR reactions, to allow for greater or lesser degrees of nucleotide sequence similarity between the degenerate primers and the corresponding sequences in the DNA. After successful amplification of a segment of the sequence encoding a BCMP, that segment may be molecularly cloned and sequenced, and utilized as a probe to isolate a complete genomic clone. This, in turn, will permit the determination of the gene's complete nucleotide sequence, the analysis of its expression, and the production of its protein product for functional analysis, as described *infra.*

The gene encoding a BCMP can also be identified by mRNA selection by nucleic acid hybridization followed by *in vitro* translation. In this procedure, fragments are used to isolate complementary mRNAs by hybridization. Such DNA fragments may represent available, purified DNA encoding a BCMP of another species *(e.g.,* mouse, human). Immunoprecipitation analysis or functional assays *(e.g.,* aggregation ability *in vitro;* binding to receptor) of the *in vitro* translation products of the isolated products of the isolated mRNAs identifies the mRNA and, therefore, the complementary DNA fragments that contain the desired sequences. In addition, specific mRNAs may be selected by adsorption of polysomes isolated from cells to immobilized antibodies that specifically recognize a BCMP. A radiolabeled cDNA encoding a BCMP can be synthesized using the selected mRNA (from the adsorbed polysomes) as a template. The radiolabeled mRNA or cDNA may then be used as a probe to identify the DNA fragments encoding a BCMP from among other genomic DNA fragments.

Alternatives to isolating genomic DNA encoding a BCMP include, but are not limited to, chemically synthesizing the gene sequence itself from a known sequence or making cDNA to the mRNA which encodes the BCMP. For example, RNA for cDNA cloning of the gene encoding a BCMP can be isolated from cells which express the BCMP. Those skilled in the art will understand from the present description that other methods may be used.

Any suitable eukaryotic cell can serve as the nucleic acid source for the molecular cloning of the gene encoding a BCMP. The nucleic acid sequences encoding the BCMP can be isolated from vertebrate, mammalian, primate, human, porcine, bovine, feline, avian, equine, canine or murine sources. The DNA may be obtained by standard procedures known in the art from cloned DNA (*e.g.,* a DNA "library"), by chemical synthesis, by cDNA cloning, or by the cloning of genomic DNA, or fragments thereof, purified from the desired cell. (See, e.g., Sambrook et al., 1989, Molecular Cloning, A Laboratory Manual, 2^{nd} Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II.) Clones derived from genomic DNA may contain regulatory and intron DNA regions in addition to coding regions; clones derived from cDNA will contain only exon sequences.

The identified and isolated gene or cDNA can then be inserted into any suitable cloning vector. A large number of vector-host systems known in the art may be used. As those skilled in the art will appreciate, the only limitation is that the vector system chosen be compatible with the host cell used. Such vectors include, but are not limited to, bacteriophages such as lambda derivatives, plasmids such as PBR322 or pUC plasmid derivatives or the Bluescript vector (Stratagene) or modified viruses such as adenoviruses, adeno-associated viruses or retroviruses. The insertion into a cloning vector can be accomplished, for example, by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. In an alternative method, the cleaved vector and the gene encoding a BCMP may be modified by homopolymeric tailing. Recombinant molecules can be introduced into host cells via transformation, transfection, infection, electroporation, etc., so that many copies of the gene sequence are generated.

In specific embodiments, transformation of host cells with recombinant DNA molecules that incorporate the isolated gene encoding the BCMP, cDNA, or synthesized DNA sequence enables generation of multiple copies of the gene. Thus, the gene may be obtained in large quantities by growing transformants, isolating the recombinant DNA molecules from the transformants and, when necessary, retrieving the inserted gene from the isolated recombinant DNA.

The nucleotide sequences include nucleotide sequences encoding amino acid sequences with substantially the same amino acid sequences as native BCMPs, nucleotide sequences encoding amino acid sequences with functionally equivalent amino acids, nucleotide sequences encoding BCMPs, fragments of BCMP, BCMP-related polypeptides, or fragments of BCMP-related polypeptides.

In a specific embodiment, an isolated nucleic acid molecule encoding a BCMP-related polypeptide can be created by introducing one or more nucleotide substitutions, additions or deletions into the nucleotide sequence of a BCMP such that one or more amino acid substitutions, additions or deletions are introduced into the encoded protein. Standard techniques known to those of skill in the art can be used to introduce mutations, including, for example, site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a side chain with a similar charge. Families of amino acid residues having side chains with similar charges have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity. Following mutagenesis, the encoded protein can be expressed and the activity of the protein can be determined.

### Expression of DNA Encoding BCMPs

The nucleotide sequence coding for a BCMP, a BCMP analog, a BCMP-related peptide, or a fragment or other derivative of any of the foregoing can be inserted into an appropriate expression vector, *i.e.,* a vector which contains the necessary elements for the transcription and translation of the inserted protein-coding sequence. The necessary transcriptional and translational signals can also be supplied by the native gene encoding the BCMP or its flanking regions, or the native gene encoding the BCMP-related polypeptide or its flanking regions. A variety of host-vector systems may be utilized in the present invention to express the protein-coding sequence. These include but are not limited to mammalian cell systems infected with virus (*e:g.,* vaccinia virus, adenovirus, etc.); insect cell systems infected with virus (*e.g.,* baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used. In specific embodiments, a nucleotide sequence encoding a human gene (or a nucleotide sequence encoding a functionally active portion of a human BCMP) is expressed. In yet another embodiment, a fragment of a BCMP comprising a domain of the BCMP is expressed.

Any of the methods previously described for the insertion of DNA fragments into a vector may be used to construct expression vectors containing a chimeric gene consisting of appropriate transcriptional and translational control signals and the protein coding sequences. These methods may include *in vitro* recombinant DNA and synthetic techniques and *in vivo* recombinants (genetic recombination). Expression of nucleic acid sequence encoding a BCMP or fragment thereof may be regulated by a second nucleic acid sequence so that the BCMP or fragment is expressed in a host transformed with the recombinant DNA molecule. For example, expression of a BCMP may be controlled by any promoter or enhancer element known in the art. Promoters which may be used to control the expression of the gene encoding a BCMP or a BCMP-related polypeptide include, but are not limited to, the SV40 early promoter region (Bernoist and Chambon, 1981, Nature 290:304-310), the promoter contained in the 3' long terminal repeat ofRous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42), the tetracycline (Tet) promoter (Gossen et al., 1995, Proc. Nat. Acad. Sci. USA 89:5547-5551); prokaryotic expression vectors such as the β-lactamase promoter (Villa-Kamaroff, et al., 1978, Proc. Natl. Acad. Sci. U.S.A. 75:3727-3731), or the *tac* promoter (DeBoer, et al., 1983, Proc. Natl. Acad. Sci. U.S.A. 80:21-25; see also "Useful proteins from recombinant bacteria" in Scientific American, 1980, 242:74-94); plant expression vectors comprising the nopaline synthetase promoter region (Herrera-Estrella et al., Nature 303:209-213) or the cauliflower mosaic virus 35S RNA promoter (Gardner, et al., 1981, Nucl. Acids Res. 9:2871), and the promoter of the photosynthetic enzyme ribulose biphosphate carboxylase (Herrera-Estrella et al., 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-4.95), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58), alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286); neuronal-specific enolase (NSE) which is active in neuronal cells (Morelli et al., 1999, Gen. Virol. 80:571-83); brain-derived neurotrophic factor (BDNF) gene control region which is active in neuronal cells (Tabuchi et al., 1998, Biochem. Biophysic. Res. Com. 253:818-823); glial fibrillary acidic protein (GFAP) promoter which is active in astrocytes (Gomes et al., 1999, Braz J Med Biol Res 32(5):619-631; Morelli et al., 1999, Gen. Virol. 80:571-83) and gonadotropic releasing hormone gene control region which is active in the hypothalamus (Mason et al., 1986, Science 234:1372-1378).

In a specific embodiment, a vector is used that comprises a promoter operably linked to a BCMP-encoding nucleic acid, one or more origins of replication, and, optionally, one or more selectable markers (*e.g.*, an antibiotic resistance gene).

In a specific embodiment, an expression construct is made by subcloning a BCMP or a BCMP-related polypeptide coding sequence into the *Eco*RI restriction site of each of the three pGEX vectors (Glutathione S-Transferase expression vectors; Smith and Johnson, 1988, Gene 7:31-40). This allows for the expression of the BCMP product or BCMP-related polypeptide from the subclone in the correct reading frame.

In mammalian host cells, a number of viral-based expression systems may be utilized. In cases where an adenovirus is used as an expression vector, the BCMP coding sequence or BCMP-related polypeptide coding sequence may be ligated to an adenovirus transcription/translation control complex, *e.g.*, the late promoter and tripartite leader sequence. This chimeric gene may then be inserted in the adenovirus genome by *in vitro* or *in vivo* recombination. Insertion in a non-essential region of the viral genome *(e.g.,* region E1 or E3) will result in a recombinant virus that is viable and capable of expressing the antibody molecule in infected hosts. *(e.g.,* see Logan & Shenk, 1984, Proc. Natl. Acad. Sci. USA 81:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., 1987, *Methods in Enzymol.* 153:51-544).

Expression vectors containing inserts of a gene encoding a BCMP or a BCMP-related polypeptide can be identified by three general approaches: (a) nucleic acid hybridization, (b) presence or absence of "marker" gene functions, and (c) expression of inserted sequences. In the first approach, the presence of a gene encoding a BCMP inserted in an expression vector can be detected by nucleic acid hybridization using probes comprising sequences that are homologous to an inserted gene encoding a BCMP. In the second approach, the recombinant vector/host system can be identified and selected based upon the presence or absence of certain "marker" gene functions (*e.g.*, thymidine kinase activity, resistance to antibiotics, transformation phenotype, occlusion body formation in baculovirus, etc.) caused by the insertion of a gene encoding a BCMP in the vector. For example, if the gene encoding the BCMP is inserted within the marker gene sequence of the vector, recombinants containing the gene encoding the BCMP insert can be identified by the absence of the marker gene function. In the third approach, recombinant expression vectors can be identified by assaying the gene product *(i.e.,* BCMP) expressed by the recombinant. Such assays can be based, for example, on the physical or functional properties of the BCMP in *in vitro* assay systems, *e.g.*, binding with anti-BCMP antibody.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered BCMP or BCMP-related polypeptide may be controlled. Furthermore, different host cells have characteristic and specific mechanisms for the translational and post-translational processing and modification (*e.g.,* glycosylation, phosphorylation of proteins). Appropriate cell lines or host systems can be chosen to ensure the desired modification and processing of the foreign protein expressed. For example, expression in a bacterial system will produce an unglycosylated product and expression in yeast will produce a glycosylated product. Eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, and WI38. Furthermore, different vector/host expression systems may effect processing reactions to different extents.

For long-term, high-yield production of recombinant proteins, stable expression is preferred. For example, cell lines which stably express the differentially expressed or pathway gene protein may be engineered. Rather than using expression vectors which contain viral origins of replication, host cells can be transformed with DNA controlled by appropriate expression control elements (*e.g*., promoter, enhancer, sequences, transcription terminators, polyadenylation sites, etc.), and a selectable marker. Following the introduction of the foreign DNA, engineered cells may be allowed to grow for 1-2 days in an enriched medium, and then are switched to a selective medium. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the differentially expressed or pathway gene protein. Such engineered cell lines may be particularly useful in screening and evaluation of compounds that affect the endogenous activity of the differentially expressed or pathway gene protein.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk-, hgprt- or aprt-cells, respectively. Also, antimetabolite resistance can be used as the basis of selection for dhfr, which confers resistance to methotrexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147) genes.

In other specific embodiments, the BCMP, fragment, analog, or derivative may be expressed as a fusion, or chimeric protein product (comprising the protein, fragment, analog, or derivative joined via a peptide bond to a heterologous protein sequence). For example, the polypeptides may be fused with the constant domain of immunoglobulins (IgA, IgE, IgG, IgM), or portions thereof (CH1, CH2, CH3, or any combination thereof and portions thereof) resulting in chimeric polypeptides. Such fusion proteins may facilitate purification, increase half-life *in vivo*, and enhance the delivery of an antigen across an epithelial barrier to the immune system. An increase in the half-life *in vivo* and facilitated purification has been shown for chimeric proteins consisting of the first two domains of the human CD4-polypeptide and various domains of the constant regions of the heavy or light chains of mammalian immunoglobulins. See, *e.g.,* EP 394,827; Traunecker *et al., Nature,* 331:84-86 (1988). Enhanced delivery of an antigen across the epithelial barrier to the immune system has been demonstrated for antigens (*e.g.,* insulin) conjugated to an FcRn binding partner such as IgG or Fc fragments (see, *e.g.,* PCT publications WO 96/22024 and WO 99/04813).

Nucleic acids encoding a BCMP, a fragment of a BCMP, a BCMP-related polypeptide, or a fragment of a BCMP-related polypeptide can be fused to an epitope tag (*e.g.,* the hemagglutinin ("HA") tag or flag tag) to aid in detection and purification of the expressed polypeptide. For example, a system described by Janknecht *et al.* allows for the ready purification of non-denatured fusion proteins expressed in human cell lines (Janknecht *et al.,* 1991, *Proc. Natl. Acad. Sci.* USA 88:8972-897).

Fusion proteins can be made by ligating the appropriate nucleic acid sequences encoding the desired amino acid sequences to each other by methods known in the art, in the proper coding frame, and expressing the chimeric product by methods commonly known in the art. Alternatively, a fusion protein may be made by protein synthetic techniques, *e.g.,* by use of a peptide synthesizer.

Both cDNA and genomic sequences can be cloned and expressed.

### Domain Structure of BCMPs

Domains of some BCMPs are known in the art and have been described in the scientific literature. Moreover, domains of a BCMP can be identified using techniques known to those of skill in the art. For example, one or more domains of a BCMP can be identified by using one or more of the following programs: ProDom, TMpred, and SAPS. ProDom compares the amino acid sequence of a polypeptide to a database of compiled domains (see, e.g., http://www.toulouse.inra.fr/prodom.html; Corpet F., Gouzy J. & Kahn D., 1999, Nucleic Acids Res., 27:263-267). TMpred predicts membrane-spanning regions of a polypeptide and their orientation. This program uses an algorithm that is based on the statistical analysis of TMbase, a database of naturally occuring transmembrane proteins (see, e.g., http://www.ch.embnet.org/software/TMPRBD_form.html: Hofmann & Stoffel. (1993) "TMbase - A database of membrane spanning proteins segments." Biol. Chem. Hoppe-Seyler 347,166). The SAPS program analyzes polypeptides for statistically significant features like charge-clusters, repeats, hydrophobic regions, compositional domains (see, e.g., Brendel et al., 1992, Proc. Natl. Acad. Sci. USA 89: 2002-2006). Thus, based on the present description, the skilled artisan can identify domains of a BCMP having enzymatic or binding activity, and further can identify nucleotide sequences encoding such domains. These nucleotide sequences can then be used for recombinant expression of a BCMP fragment that retains the enzymatic or binding activity of the BCMP.

Based on the present description, the skilled artisan can identify domains of a BCMP having enzymatic or binding activity, and further can identify nucleotide sequences encoding such domains. These nucleotide sequences can then be used for recombinant expression of BCMP fragments that retain the enzymatic or binding activity of the BCMP.

In one embodiment, a BCMP has an amino acid sequence sufficiently similar to an identified domain of a known polypeptide. As used herein, the term "sufficiently similar" refers to a first amino acid or nucleotide sequence which contains a sufficient number of identical or equivalent (e.g., with a similar side chain) amino acid residues or nucleotides to a second amino acid or nucleotide sequence such that the first and second amino acid or nucleotide sequences have or encode a common structural domain or common functional activity or both.

A BCMP domain can be assessed for its function using techniques well known to those of skill in the art. For example, a domain can be assessed for its kinase activity or for its ability to bind to DNA using techniques known to the skilled artisan. Kinase activity can be assessed, for example, by measuring the ability of a polypeptide to phosphorylate a substrate. DNA binding activity can be assessed, for example, by measuring the ability of a polypeptide to bind to a DNA binding element in a electromobility shift assay.

### Production of Antibodies to BCMPs

According to the invention a BCMP, BCMP analog, BCMP-related protein or a fragment or derivative of any of the foregoing may be used as an immunogen to generate antibodies which immunospecifically bind such an immunogen. Such immunogens can be isolated by any convenient means, including the methods described-above. Antibodies include, but are not limited to polyclonal, monoclonal, bispecific, humanized or chimeric antibodies, single chain antibodies, Fab fragments and F(ab') fragments, fragments produced by a Fab expression library, anti-idiotypic (anti-Id) antibodies, and epitope-binding fragments of any of the above. The term "antibody" as used herein refers to immunoglobulin molecules and immunologically active portions of immunoglobulin molecules, *i.e.,* molecules that contain an antigen binding site that specifically binds an antigen. The immunoglobulin molecules can be of any class (e.g., IgG, IgE, IgM, IgD and IgA) or subclass of immunoglobulin molecule.

In one embodiment, antibodies that recognize gene products of genes encoding BCMPs are publicly available. In another embodiment, methods known to those skilled in the art are used to produce antibodies that recognize a BCMP, a BCMP analog, a BCMP-related polypeptide, or a fragment or derivative of any of the foregoing.

In one embodiment, antibodies to a specific domain of a BCMP are produced. In a specific embodiment, hydrophilic fragments of a BCMP are used as immunogens for antibody production.

In the production of antibodies, screening for the desired antibody can be accomplished by techniques known in the art, *e.g.* ELISA (enzyme-linked immunosorbent assay). For example, to select antibodies which recognize a specific domain of a BCMP, one may assay generated hybridomas for a product which binds to a BCMP fragment containing such domain. For selection of an antibody that specifically binds a first BCMP homolog but which does not specifically bind to (or binds less avidly to) a second BCMP homolog, one can select on the basis of positive binding to the first BCMP homolog and a lack of binding to (or reduced binding to) the second BCMP homolog. Similarly, for selection of an antibody that specifically binds a BCMP but which does not specifically bind to (or binds less avidly to) a different isoform of the same protein (such as a different glycoform having the same core peptide as the BCMP), one can select on the basis of positive binding to the BCMP and a lack of binding to (or reduced binding to) the different isoform *(e.g.*, a different glycoform). Thus, the present invention provides the use of an antibody (preferably a monoclonal antibody) that binds with greater affinity (preferably at least 2-fold, more preferably at least 5-fold still more preferably at least 10-fold greater affinity) to a BCMP than to a different isoform or isoforms (*e.g*., glycoforms) of the BCMP.

Polyclonal antibodies which may be used in the methods of the invention are heterogeneous populations of antibody molecules derived from the sera of immunized animals. Unfractionated immune serum can also be used. Various procedures known in the art may be used for the production of polyclonal antibodies to a BCMP, a fragment of a BCMP, a BCMP-related polypeptide, or a fragment of a BCMP-related polypeptide.. In a particular embodiment, rabbit polyclonal antibodies to an epitope of a BCMP or a BCMP-related polypeptide can be obtained. For example, for the production of polyclonal or monoclonal antibodies, various host animals can be immunized by injection with the native or a synthetic (*e.g.,* recombinant) version of a BCMP, a fragment of a BCMP, a BCMP-related polypeptide, or a fragment of a BCMP-related polypeptide, including but not limited to rabbits, mice, rats, etc. The Preferred Technology described herein provides isolated BCMPs suitable for such immunization. If the BCMP is purified by gel electrophoresis, the BCMP can be used for immunization with or without prior extraction from the polyacrylamide gel. Various adjuvants may be used to enhance the immunological response, depending on the host species, including, but not limited to, complete or incomplete Freund's adjuvant, a mineral gel such as aluminum hydroxide, surface active substance such as lysolecithin, pluronic polyol, a polyanion, a peptide, an oil emulsion, keyhole limpet hemocyanin, dinitrophenol, and an adjuvant such as BCG (bacille Calmette-Guerin) or corynebacterium parvum. Additional adjuvants are also well known in the art.

For preparation of monoclonal antibodies (mAbs) directed toward a BCMP, a fragment of a BCMP, a BCMP-related polypeptide, or a fragment of a BCMP-related polypeptide, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs may be cultivated *in vitro* or *in vivo*. In an additional embodiment, monoclonal antibodies can be produced in germ-free animals utilizing known technology (PCT/US90/02545).

The monoclonal antibodies include but are not limited to human monoclonal antibodies and chimeric monoclonal antibodies *(e.g.,* human-mouse chimeras). A chimeric antibody is a molecule in which different portions are derived from different animal species, such as those having a human immunoglobulin constant region and a variable region derived from a murine mAb. (See, e.g., Cabilly et al., U.S. Patent No. 4,816,567; and Boss et al., U.S. Patent No. 4,816397). Humanized antibodies are antibody molecules from non-human species having one or more complementarily determining regions (CDRs) from the non-human species and a framework region from a human immunoglobulin molecule. (See, e.g., Queen, U.S. Patent No. 5,585,089).

Chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, Bio/Techniques 4:214; U.S. Patent 5,225,539; Jones et al., 1986, Nature 321:552-525; Verhoeyan et al. (1988) Science 239:1534; and Beidler et al., 1988, J. Immunol. 141:4053-4060.

Completely human antibodies are particularly desirable for therapeutic treatment of human subjects. Such antibodies can be produced using transgenic mice which are incapable of expressing endogenous immunoglobulin heavy and light chain genes, but which can express human heavy and light chain genes. The transgenic mice are immunized in the normal fashion with a selected antigen, *e.g.,* all or a portion of a BCMP. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g.,* U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection." In this approach a selected non-human monoclonal antibody, *e.g.,* a mouse antibody, is used to guide the selection of a completely human antibody recognizing the same epitope. (Jespers et al. (1994) Bio/technology 12:899-903).

The antibodies can also be generated using various phage display methods known in the art. In phage display methods, functional antibody domains are displayed on the surface of phage particles which carry the polynucleotide sequences encoding them. In a particular, such phage can be utilized to display antigen binding domains expressed from a repertoire or combinatorial antibody library (*e.g.,* human or murine). Phage expressing an antigen binding domain that binds the antigen of interest can be selected or identified with antigen, *e.g.*, using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Phage display methods that can be used to make the antibodies include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT Application No. PCT/GB91/01134; PCT Publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969, 108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')₂ fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988).

Examples of techniques which can be used to produce single-chain Fvs and antibodies include those described in U.S. Patents 4,946,778 and 5,258,498; Huston *et al., Methods in Enzymology* 203:46-88 (1991); Shu *et al., PNAS* 90:7995-7999 (1993); and Skerra *et al., Science* 240:1038-1040 (1988).

The invention further provides for the use of bispecific antibodies, which can be made by methods known in the art. Traditional production of full length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, published 13 May 1993, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published March 3,1994. For further details for generating bispecific antibodies see, for example, Suresh et al., Methods in Enzymology, 1986, 121:210.

The invention provides the use of functionally active fragments, derivatives or analogs of the anti-BCMP immunoglobulin molecules. Functionally active means that the fragment, derivative or analog is able to elicit anti-anti-idiotype antibodies (*i.e*., tertiary antibodies) that recognize the same antigen that is recognized by the antibody from which the fragment, derivative or analog is derived. Specifically, in a preferred embodiment the antigenicity of the idiotype of the immunoglobulin molecule may be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art.

The present invention provides the use of antibody fragments such as, but not limited to, F(ab')₂ fragments and Fab fragments. Antibody fragments which recognize specific epitopes may be generated by known techniques. F(ab')₂ fragments consist of the variable region, the light chain constant region and the CH1 domain of the heavy chain and are generated by pepsin digestion of the antibody molecule. Fab fragments are generated by reducing the disulfide bridges of the F(ab')₂ fragments. The invention also provides the use of heavy chain and light chain dimers of the antibodies or any minimal fragment thereof such as Fvs or single chain antibodies (SCAs) (e.g., as described in U.S. Patent 4,946,778; Bird, 1988, Science 242:423-42; Huston et al., 1988, Proc. Natl. Acad. Sci. USA 85:5879-5883; and Ward et al., 1989, Nature 334:544-54), or any other molecule with the same specificity as the antibody. Single chain antibodies are formed by linking the heavy and light chain fragments of the Fv region via an amino acid bridge, resulting in a single chain polypeptide. Techniques for the assembly of functional Fv fragments in *E. coli* may be used (Skerra et al., 1988, Science 242:1038-1041).

In other embodiments, the invention provides the use of fusion proteins of the immunoglobulins (or functionally active fragments thereof), for example in which the immunoglobulin is fused via a covalent bond (*e.g.,* a peptide bond), at either the N-terminus or the C-terminus to an amino acid sequence of another protein (or portion thereof, preferably at least 10, 20 or 50 amino acid portion of the protein) that is not the immunoglobulin. Preferably the immunoglobulin, or fragment thereof, is covalently linked to the other protein at the N-terminus of the constant domain. As stated above, such fusion proteins may facilitate purification, increase half-life *in vivo*, and enhance the delivery of an antigen across an epithelial barrier to the immune system.

The immunoglobulins useful in the invention include analogs and derivatives that are either modified, i.e, by the covalent attachment of any type of molecule as long as such covalent attachment that does not impair immunospecific binding. For example, but not by way of limitation, the derivatives and analogs of the immunoglobulins include those that have been further modified, *e.g.,* by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analog or derivative may contain one or more non-classical amino acids.

The foregoing antibodies can be used in methods known in the art relating to the localization and activity of the BCMPs, *e.g.*, for imaging these proteins, measuring levels thereof in appropriate physiological samples, in diagnostic methods, etc.

### Expression Of Antibodies

The antibodies to be used according to the invention can be produced by any method known in the art for the synthesis of antibodies, in particular, by chemical synthesis or by recombinant expression, and are preferably produced by recombinant expression techniques.

Recombinant expression of antibodies, or fragments, derivatives or analogs thereof, requires construction of a nucleic acid that encodes the antibody. If the nucleotide sequence of the antibody is known, a nucleic acid encoding the antibody may be assembled from chemically synthesized oligonucleotides (*e.g.*, as described in Kutmeier et al., 1994, BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (e.g., an antibody cDNA library, or cDNA library generated from any tissue or cells expressing the antibody) by PCR amplification using synthetic primers hybridizable to the 3' and 5' ends of the sequence or by cloning using an oligonucleotide probe specific for the particular gene sequence.

If an antibody molecule that specifically recognizes a particular antigen is not available (or a source for a cDNA library for cloning a nucleic acid encoding such an antibody), antibodies specific for a particular antigen may be generated by any method known in the art, for example, by immunizing an animal, such as a rabbit, to generate polyclonal antibodies or, more preferably, by generating monoclonal antibodies. Alternatively, a clone encoding at least the Fab portion of the antibody may be obtained by screening Fab expression libraries (*e.g.*, as described in Huse et al., 1989, *Science* 246:1275-1281) for clones of Fab fragments that bind the specific antigen or by screening antibody libraries (See, *e.g.,* Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, *e.g.,* PCT Publication WO 86/05807; PCT Publication WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulfide bond with an amino acid residue that does not contain a sulfhydyl group. Such modifications can be carried out by any method known in the art for the introduction of specific mutations or deletions in a nucleotide sequence, for example, but not limited to, chemical mutagenesis, in vitro site directed mutagenesis (Hutchinson et al., 1978, J. Biol. Chem. 253:6551), PCT based methods, etc.

In addition, techniques developed for the production of "chimeric antibodies" (Morrison et al., 1984, Proc. Natl. Acad. Sci. 81:851-855; Neuberger et al., 1984, *Nature* 312:604-608; Takeda et al., 1985, Nature 314:452-454) by splicing genes from a mouse antibody molecule of appropriate antigen specificity together with genes from a human antibody molecule of appropriate biological activity can be used. As described *supra,* a** chimeric antibody is a molecule in which different portions are derived from different animal species, such as those having a variable region derived from a murine mAb and a human antibody constant region, *e.g.,* humanized antibodies.

Once a nucleic acid encoding an antibody molecule has been obtained, the vector for the production of the antibody molecule may be produced by recombinant DNA technology using techniques well known in the art. Thus, methods for preparing the protein by expressing nucleic acid containing the antibody molecule sequences are described herein. Methods which are well known to those skilled in the art can be used to construct expression vectors containing an antibody molecule coding sequences and appropriate transcriptional and translational control signals. These methods include, for example, *in vitro* recombinant DNA techniques, synthetic techniques, and *in vivo* genetic recombination. See, for example, the techniques described in Sambrook et al. (1990, Molecular Cloning, A Laboratory Manual, 2^{nd} Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody.

The host cells used to express a recombinant antibody may be either bacterial cells such as *Escherichia coli,* or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; Cockett et al., 1990, Bio/Technology 8:2).

A variety of host-expression vector systems may be utilized to express an antibody molecule. Such host-expression systems represent vehicles by which the coding sequences of interest may be produced and subsequently purified, but also represent cells which may, when transformed or transfected with the appropriate nucleotide coding sequences, express the antibody molecule *in situ.* These include but are not limited to microorganisms such as bacteria *(e.g., E. coli, B. subtilis)* transformed with recombinant bacteriophage DNA, plasmid DNA or cosmid DNA expression vectors containing antibody coding sequences; yeast (*e.g., Saccharomyces, Pichia)* transformed with recombinant yeast expression vectors containing antibody coding sequences; insect cell systems infected with recombinant virus expression vectors (*e.g.,* baculovirus) containing the antibody coding sequences; plant cell systems infected with recombinant virus expression vectors (*e.g.,* cauliflower mosaic virus, CaMV; tobacco mosaic virus, TMV) or transformed with recombinant plasmid expression vectors (*e.g*., Ti plasmid) containing antibody coding sequences; or mammalian cell systems *(e.g.,* COS, CHO, BHK, 293, 3T3 cells) harboring recombinant expression constructs containing promoters derived from the genome of mammalian cells *(e.g.,* metallothionein promoter) or from mammalian viruses (*e.g.,* the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for the generation of pharmaceutical compositions comprising an antibody molecule, vectors which direct the expression of high levels of fusion protein products that are readily purified may be desirable. Such vectors include, but are not limited, to the *E*. *coli* expression vector pUR278 (Ruther et al., 1983, EMBO J. 2:1791), in which the antibody coding sequence may be ligated individually into the vector in frame with the *lac Z* coding region so that a fusion protein is produced; pIN vectors (Inouye & Inouye, 1985, *Nucleic Acids Res.* 13:3101-3109; Van Heeke & Schuster, 1989, J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter). In mammalian host cells, a number of viral-based expression systems (*e.g.*, an adenovirus expression system) may be utilized.

As discussed above, a host cell strain may be chosen which modulates the expression of the inserted sequences, or modifies and processes the gene product in the specific fashion desired. Such modifications (*e.g.,* glycosylation) and processing (*e.g.,* cleavage) of protein products may be important for the function of the protein.

For long-term, high-yield production of recombinant antibodies, stable expression is preferred. For example, cells lines that stably express an antibody of interest can be produced by transfecting the cells with an expression vector comprising the nucleotide sequence of the antibody and the nucleotide sequence of a selectable (*e.g.,* neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, Proc. Natl. Acad. Sci. USA 77:2197): The coding sequences for the heavy and light chains may comprise cDNA or genomic DNA.

Once the antibody molecule has been recombinantly expressed, it may be purified by any method known in the art for purification of an antibody molecule, for example, by chromatography (e.g., ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.

### Conjugated Antibodies

In a preferred embodiment, anti-BCMP antibodies or fragments thereof are conjugated to a diagnostic or therapeutic moiety. The antibodies can be used for diagnosis or to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present invention. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I,¹³¹I, ¹¹¹In and ⁹⁹Tc.

Anti-BCMP antibodies or fragments thereof can be conjugated to a therapeutic agent or drug moiety to modify a given biological response. The therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator, a thrombotic agent or an anti-angiogenic agent, *e.g.,* angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2^{nd} Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described by Segal in U.S. Patent No. 4,676,980.

An antibody with or without a therapeutic moiety conjugated to it can be used as a therapeutic that is administered alone or in combination with cytotoxic factor(s) and/or cytokine(s).

### Diagnosis Of Breast Cancer

In accordance with the present invention, test samples of breast tissue, serum, plasma or urine obtained from a subject suspected of having or known to have breast cancer can be used for diagnosis or monitoring. In one embodiment, a change in the abundance of one or more BCMPs in a test sample relative to a control sample (from a subject or subjects free from breast cancer) or a previously determined reference range indicates the presence of breast cancer. In another embodiment, the relative abundance of one or more BCMPs in a test sample compared to a control sample or a previously determined reference range indicates a subtype of breast cancer (*e.g.,* familial or sporadic breast cancer). In yet another embodiment, the relative abundance of one or more BCMPs in a test sample relative to a control sample or a previously determined reference range indicates the degree or severity of breast cancer (e.g., the likelihood for metastasis). In any of the aforesaid methods, detection of one or more BCMPs may optionally be combined with detection of one or more additional biomarkers for breast cancer. Any suitable method in the art can be employed to measure the level of BCMPs, including but not limited to the Preferred Technology described herein, kinase assays, immunoassays to detect and/or visualize the BCMPs (*e.g.,* Western blot, immunoprecipitation followed by sodium dodecyl sulfate polyacrylamide gel electrophoresis, immunocytochemistry, etc.). In cases where a BCMP has a known function, an assay for that function may be used to measure BCMP expression. In a further embodiment, a change in the abundance of mRNA encoding BCMPs in a test sample relative to a control sample or a previously determined reference range indicates the presence of breast cancer. Any suitable hybridization assay can be used to detect BCMP expression by detecting and/or visualizing mRNA encoding the BCMP (*e.g*., Northern assays, dot blots, *in situ* hybridization, etc.).

In another embodiment of the invention, labeled antibodies, derivatives and analogs thereof, which specifically bind to a BCMP can be used for diagnostic purposes to detect, diagnose, or monitor breast cancer. Preferably, breast cancer is detected in an animal, more preferably in a mammal and most preferably in a human.

### Screening Assays

The invention provides methods for identifying agents (e.g., candidate compounds or test compounds) that bind to a BCMP or have a stimulatory or inhibitory effect on the expression or activity of a BCMP. The invention also provides methods of identifying agents, candidate compounds or test compounds that bind to a BCMP-related polypeptide or a BCMP fusion protein or have a stimulatory or inhibitory effect on the expression or activity of a BCMP-related polypeptide or a BCMP fusion protein. Examples of agents, candidate compounds or test compounds include, but are not limited to, nucleic acids (*e.g.*, DNA and RNA), carbohydrates, lipids, proteins, peptides, peptidomimetics, small molecules and other drugs. Agents can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, 1997, Anticancer Drug Des. 12:145; U.S. Patent No. 5,738,996; and U.S. Patent No.5,807,683).

Examples of methods for the synthesis of molecular libraries can be found in the art, for example in: DeWitt et al., 1993, Proc. Natl. Acad. Sci. USA 90:6909; Erb et al., 1994, Proc. Natl. Acad. Sci. USA 91:11422; Zuckermann et al., 1994, J. Med. Chem. 37:2678; Cho et al., 1993, Science 261:1303; Carrell et al., 1994, Angew. Chem. Int. Ed. Engl. 33:2059; Carell et al., 1994, Angew. Chem. Int. Ed. Engl. 33:2061; and Gallop et al., 1994, J. Med. Chem. 37:1233.

Libraries of compounds may be presented, e.g., presented in solution (*e.g.,* Houghten, 1992, Bio/Techniques 13:412-421), or on beads (Lam, 1991, Nature 354:82-84), chips (Fodor, 1993, Nature 364:555-556), bacteria (U.S. Patent No. 5,223,409), spores (Patent Nos. 5,571,698; 5,403,484; and 5,223,409), plasmids (Cull et al., 1992, Proc. Natl. Acad. Sci. USA 89:1865-1869) or phage (Scott and Smith, 1990, Science 249:386-390; Devlin, 1990, Science 249:404-406; Cwirla et al., 1990, Proc. Natl. Acad. Sci. USA 87:6378-6382; and Felici, 1991, J. Mol. Biol. 222:301-310).

In one embodiment, agents that interact with (*i.e.,* bind to) a BCMP, a BCMP fragment (e.g. a functionally active fragment), a BCMP-related polypeptide, a fragment of a BCMP-related polypeptide, or a BCMP fusion protein are identified in a cell-based assay system. In accordance with this embodiment, cells expressing a BCMP, a fragment of a BCMP, a BCMP-related polypeptide, a fragment of the BCMP-related polypeptide, or a BCMP fusion protein are contacted with a candidate compound or a control compound and the ability of the candidate compound to interact with the BCMP is determined. If desired, this assay may be used to screen a plurality (e.g. a library) of candidate compounds. The cell, for example, can be of prokaryotic origin *(e.g., E. coli)* or eukaryotic origin (*e.g.,* yeast or mammalian). Further, the cells can express the BCMP, fragment of the BCMP, BCMP-related polypeptide, a fragment of the BCMP-related polypeptide, or a BCMP fusion protein endogenously or be genetically engineered to express the BCMP, fragment of the BCMP, BCMP-related polypeptide, a fragment of the BCMP-related polypeptide, or a BCMP fusion protein. In certain instances, the BCMP, fragment of the BCMP, BCMP-related polypeptide, a fragment of the BCMP-related polypeptide, or a BCMP fusion protein or the candidate compound is labeled, for example with a radioactive label (such as ³²p, ³⁵S, and ¹²⁵I) or a fluorescent label (such as fluorescein isothiocyanate, rhodamine, phycoerythrin, phycocyanin, allophycocyanin, o-phthaldehyde or fluorescamine) to enable detection of an interaction between a BCMP and a candidate compound. The ability of the candidate compound to interact directly or indirectly with a BCMP, a fragment of a BCMP, a BCMP-related polypeptide, a fragment of a BCMP-related polypeptide, or a BCMP fusion protein can be determined by methods known to those of skill in the art. For example, the interaction between a candidate compound and a BCMP, a BCMP-related polypeptide, a fragment of a BCMP-related polypeptide, or a BCMP fusion protein can be determined by flow cytometry, a scintillation assay, immunoprecipitation or western blot analysis.

In another embodiment, agents that interact with (*i.e.,* bind to) a BCMP, a BCMP fragment (e.g., a functionally active fragment), a BCMP-related polypeptide, a fragment of a BCMP-related polypeptide, or a BCMP fusion protein are identified in a cell-free assay system. In accordance with this embodiment, a native or recombinant BCMP or fragment thereof, or a native or recombinant BCMP-related polypeptide or fragment thereof, or a BCMP-fusion protein or fragment thereof, is contacted with a candidate compound or a control compound and the ability of the candidate compound to interact with the BCMP or BCMP-related polypeptide, or BCMP fusion protein is determined. If desired, this assay may be used to screen a plurality (e.g. a library) of candidate compounds. Preferably, the BCMP, BCMP fragment, BCMP-related polypeptide, a fragment of a BCMP-related polypeptide, or a BCMP-fusion protein is first immobilized, by, for example, contacting the BCMP, BCMP fragment, BCMP-related polypeptide, a fragment of a BCMP-related polypeptide, or a BCMP fusion protein with an immobilized antibody which specifically recognizes and binds it, or by contacting a purified preparation of the BCMP, BCMP fragment, BCMP-related polypeptide, fragment of a BCMP-related polypeptide, or a BCMP fusion protein with a surface designed to bind proteins. The BCMP, BCMP fragment, BCMP-related polypeptide, a fragment of a BCMP-related polypeptide, or a BCMP fusion protein may be partially or completely purified (*e.g.,* partially or completely free of other polypeptides) or part of a cell lysate. Further, the BCMP, BCMP fragment, BCMP-related polypeptide, fragment of a BCMP-related polypeptide may be a fusion protein comprising the BCMP or a biologically active portion thereof, or BCMP-related polypeptide and a domain such as glutathionine-S-transferase. Alternatively, the BCMP, BCMP fragment, BCMP-related polypeptide, fragment of a BCMP-related polypeptide or BCMP fusion protein can be biotinylated using techniques well known to those of skill in the art *(e.g.,* biotinylation kit, Pierce Chemicals; Rockford, IL). The ability of the candidate compound to interact with a BCMP, BCMP fragment, BCMP-related polypeptide, a fragment of a BCMP-related polypeptide, or a BCMP fusion protein can be determined by methods known to those of skill in the art.

In another embodiment, a cell-based assay system is used to identify agents that bind to or modulate the activity of a protein, such as an enzyme, or a biologically active portion thereof, which is responsible for the production or degradation of a BCMP or is responsible for the post-translational modification of a BCMP. In a primary screen, a plurality (e.g., a library) of compounds are contacted with cells that naturally or recombinantly express: (i) a BCMP, an isoform of a BCMP, a BCMP homolog, a BCMP-related polypeptide, a BCMP fusion protein, or a biologically active fragment of any of the foregoing; and (ii) a protein that is responsible for processing of the BCMP, BCMP isoform, BCMP homolog, BCMP-related polypeptide, BCMP fusion protein, or fragment in order to identify compounds that modulate the production, degradation, or post-translational modification of the BCMP, BCMP isoform, BCMP homolog, BCMP-related polypeptide, BCMP fusion protein or fragment. If desired, compounds identified in the primary screen can then be assayed in a secondary screen against cells naturally or recombinantly expressing the specific BCMPs of interest. The ability of the candidate compound to modulate the production, degradation or post-translational modification of a BCMP, isoform, homolog, BCMP-related polypeptide, or BCMP fusion protein can be determined by methods known to those of skill in the art, including without limitation, flow cytometry, a scintillation assay, immunoprecipitation and western blot analysis.

In another embodiment, agents that competitively interact with (*i.e.*, bind to) a BCMP, BCMP fragment, BCMP-related polypeptide, a fragment of a BCMP-related polypeptide, or a BCMP fusion protein are identified in a competitive binding assay. In accordance with this embodiment, cells expressing a BCMP, BCMP fragment, BCMP-related polypeptide, a fragment of a BCMP-related polypeptide, or a BCMP fusion protein are contacted with a candidate compound and a compound known to interact with the BCMP, BCMP fragment, BCMP-related polypeptide, a fragment of a BCMP-related polypeptide or a BCMP fusion protein; the ability of the candidate compound to preferentially interact with the BCMP, BCMP fragment, BCMP-related polypeptide, fragment of a BCMP-related polypeptide, or a BCMP fusion protein is then determined. Alternatively, agents that preferentially interact with *(i.e.,* bind to) a BCMP, BCMP fragment, BCMP-related polypeptide or fragment of a BCMP-related polypeptide are identified in a cell-free assay system by contacting a BCMP, BCMP fragment, BCMP-related polypeptide, fragment of a BCMP-related polypeptide, or a BCMP fusion protein with a candidate compound and a compound known to interact with the BCMP, BCMP-related polypeptide or BCMP fusion protein. As stated above, the ability of the candidate compound to interact with a BCMP, BCMP fragment, BCMP-related polypeptide, a fragment of a BCMP-related polypeptide, or a BCMP fusion protein can be determined by methods known to those of skill in the art. These assays, whether cell-based or cell-free, can be used to screen a plurality (e.g., a library) of candidate compounds.

In another embodiment, agents that modulate (*i.e*., upregulate or downregulate) the expression or activity of a BCMP, or a BCMP-related polypeptide are identified by contacting cells (*e.g.*, cells of prokaryotic origin or eukaryotic origin) expressing the BCMP, or BCMP-related polypeptide with a candidate compound or a control compound (e.g., phosphate buffered saline (PBS)) and determining the expression of the BCMP, BCMP-related polypeptide, or BCMP fusion protein, mRNA encoding the BCMP, or mRNA encoding the BCMP-related polypeptide. The level of expression of a selected BCMP, BCMP-related polypeptide, mRNA encoding the BCMP, or mRNA encoding the BCMP-related polypeptide in the presence of the candidate compound is compared to the level of expression of the BCMP, BCMP-related polypeptide, mRNA encoding the BCMP, or mRNA encoding the BCMP-related polypeptide in the absence of the candidate compound (*e.g.*, in the presence of a control compound). The candidate compound can then be identified as a modulator of the expression of the BCMP, or the BCMP-related polypeptide based on this comparison. For example, when expression of the BCMP or mRNA is significantly greater in the presence of the candidate compound than in its absence, the candidate compound is identified as a stimulator of expression of the BCMP or mRNA. Alternatively, when expression of the BCMP or mRNA is significantly less in the presence of the candidate compound than in its absence, the candidate compound is identified as an inhibitor of the expression of the BCMP or mRNA. The level of expression of a BCMP or the mRNA that encodes it can be determined by methods known to those of skill in the art. For example, mRNA expression can be assessed by Northern blot analysis or RT-PCR, and protein levels can be assessed by western blot analysis.

In another embodiment, agents that modulate the activity of a BCMP or a BCMP-related polypeptide are identified by contacting a preparation containing the BCMP or BCMP-related polypeptide or cells (*e.g.*, prokaryotic or eukaryotic cells) expressing the BCMP or BCMP-related polypeptide with a test compound or a control compound and determining the ability of the test compound to modulate (*e.g.,* stimulate or inhibit) the activity of the BCMP or BCMP-related polypeptide. The activity of a BCMP or a BCMP-related-polypeptide can be assessed by detecting induction of a cellular signal transduction pathway of the BCMP or BCMP-related polypeptide (*e.g.* , intracellular Ca²⁺, diacylglycerol, IP3, etc.), detecting catalytic or enzymatic activity of the target on a suitable substrate, detecting the induction of a reporter gene (*e.g.*, a regulatory element that is responsive to a BCMP or a BCMP-related polypeptide and is operably linked to a nucleic acid encoding a detectable marker, *e.g.*, luciferase), or detecting a cellular response, for example, cellular differentiation, or cell proliferation. Based on the present description, techniques known to those of skill in the art can be used for measuring these activities (see, *e.g.,* U.S. Patent No. 5,401,639, which is incorporated herein by reference). The candidate compound can then be identified as a modulator of the activity of a BCMP or a BCMP-related polypeptide by comparing the effects of the candidate compound to the control compound. Suitable control compounds include phosphate buffered saline (PBS) and normal saline (NS)..

In another embodiment, agents that modulate (i.e., upregulate or downregulate) the expression, activity or both the expression and activity of a BCMP or BCMP-related polypeptide are identified in an animal model. Examples of suitable animals include, but are not limited to, mice, rats, rabbits, monkeys, guinea pigs, dogs and cats. Preferably, the animal used represent a model of breast cancer *(e.g.,* xenografts of human breast cancer cell lines such as MDA-MB-345 in oestrogen-deprived Severe Combined Immunodeficient (SCID) mice, Eccles et al. 1994 Cell Biophysics 24/25, 279). These can be utilized to test compounds that modulate BCMP levels, since the pathology exhibited in these models is similar to that of breast cancer. In accordance with this embodiment, the test compound or a control compound is administered (*e.g.*, orally, rectally or parenterally such as intraperitoneally or intravenously) to a suitable animal and the effect on the expression, activity or both expression and activity of the BCMP or BCMP-related polypeptide is determined. Changes in the expression of a BCMP or BCMP-related polypeptide can be assessed by the methods outlined above.

In yet another embodiment, a BCMP or BCMP-related polypeptide is used as a "bait protein" in a two-hybrid assay or three hybrid assay to identify other proteins that bind to or interact with a BCMP or BCMP-related polypeptide *(see, e.g.,* U.S. Patent No. 5,283,317; Zervos et al. (1993) Cell 72:223-232; Madura et al. (1993) J. Biol. Chem. 268:12046-12054; Bartel et al. (1993) Bio/Techniques 14:920-924; Iwabuchi et al. (1993) Oncogene 8:1693-1696; and PCT Publication No. WO 94/10300). As those skilled in the art will appreciate, such binding proteins are also likely to be involved in the propagation of signals by the BCMPs of the invention as, for example, upstream or downstream elements of a signaling pathway involving the BCMPs.

Agents identified by the above-described screening assays can be used for treatments as described herein. In addition, the invention also provides the use of an agent which interacts with, or modulates the activity of, one or more BCMPs of the invention in the manufacture of a medicament for the treatment of breast cancer.

### Therapeutic Use of BCMPs

The invention is useful for treatment or prevention of various diseases and disorders by administration of a therapeutic compound. Such compounds include but are not limited to: BCMPs, BCMP analogs, BCMP-related polypeptides and derivatives (including fragments) thereof; antibodies to the foregoing; nucleic acids encoding BCMPs, BCMP analogs, BCMP-related polypeptides and fragments thereof; antisense nucleic acids to a gene encoding a BCMP or BCMP-related polypeptide; and modulator (e.g., agonists and antagonists) of a gene encoding a BCMP or BCMP-related polypeptide. The present invention is based on the identification of genes encoding BCMPs involved in breast cancer. Breast cancer can be treated (e.g. to ameliorate symptoms or to retard onset or progression) or prevented by administration of a therapeutic compound that promotes function or expression of one or more BCMPs that are decreased in the breast tissue of subjects having breast cancer, or by administration of a therapeutic compound that reduces function or expression of one or more BCMPs that are increased in the breast tissue of subjects having breast cancer.

In one embodiment, one or more antibodies each specifically binding to a BCMP are administered alone or in combination with one or more additional therapeutic compounds or treatments. Examples of such therapeutic compounds or treatments include, but are not limited to, taxol, cyclophosphamide, tamoxifen, and doxorubacin.

Preferably, a biological product such as an antibody is allogeneic to the subject to which it is administered. In a preferred embodiment, a human BCMP or a human BCMP-related polypeptide, a nucleotide sequence encoding a human BCMP or a human BCMP-related polypeptide, or an antibody to a human BCMP or a human BCMP-related polypeptide, is administered to a human subject for therapy (e.g. to ameliorate symptoms or to retard onset or progression) or prophylaxis.

### Treatment And Prevention of Breast Cancer

Breast cancer is treated or prevented by administration to a subj ect suspected of having or known to have breast cancer or to be at risk of developing breast cancer of a compound that modulates (*i.e*., increases or decreases) the level or activity (*i.e.*, function) of one or more BCMPs that are differentially present in the breast tissue of subjects having breast cancer compared with breast tissue of subjects free from breast cancer. In one embodiment, breast cancer is treated or prevented by administering to a subject suspected of having or known to have breast cancer or to be at risk of developing breast cancer a compound that upregulates (*i.e.*, increases) the level or activity (*i.e.*, function) of one or more BCMPs that are decreased in the breast tissue of subjects having breast cancer. In another embodiment, a compound is administered that downregulates the level or activity *(i.e.,* function) of one or more BCMPs that are increased in the breast tissue of subjects having breast cancer. Examples of such a compound include but are not limited to: BCMPs, BCMP fragments and BCMP-related polypeptides; nucleic acids encoding a BCMP, a BCMP fragment and a BCMP-related polypeptide (*e.g.,* for use in gene therapy); and, for those BCMPs or BCMP-related polypeptides with enzymatic activity, compounds or molecules known to modulate that enzymatic activity. Other compounds that can be used, *e.g.,* BCMP agonists, can be identified using *in vitro* assays.

Breast cancer is also treated or prevented by administration to a subject suspected of having or known to have breast cancer or to be at risk of developing breast cancer of a compound that downregulates the level or activity of one or more BCMPs that are increased in the breast tissue of subjects having breast cancer. In another embodiment, a compound is administered that upregulates the level or activity of one or more BCMPs that are decreased in the breast tissue of subjects having breast cancer. Examples of such a compound include, but are not limited to, BCMP antisense oligonucleotides, ribozymes, antibodies directed against BCMPs, and compounds that inhibit the enzymatic activity of a BCMP. Other useful compounds *e.g.,* BCMP antagonists and small molecule BCMP antagonists, can be identified using *in vitro* assays.

In a preferred embodiment, therapy or prophylaxis is tailored to the needs of an individual subject. Thus, in specific embodiments, compounds that promote the level or function of one or more BCMPs are therapeutically or prophylactically administered to a subject suspected of having or known to have breast cancer, in whom the levels or functions of said one or more BCMPs are absent or are decreased relative to a control or normal reference range. In further embodiments, compounds that promote the level or function of one or more BCMPs are therapeutically or prophylactically administered to a subject suspected of having or known to have breast cancer in whom the levels or functions of said one or more BCMPs are increased relative to a control or to a reference range. In further embodiments, compounds that decrease the level or function of one or more BCMPs are therapeutically or prophylactically administered to a subject suspected of having or known to have breast cancer in whom the levels or functions of said one or more BCMPs are increased relative to a control or to a reference range. In further embodiments, compounds that decrease the level or function of one or more BCMPs are therapeutically or prophylactically administered to a subject suspected of having or known to have breast cancer in whom the levels or functions of said one or more BCMPs are decreased relative to a control or to a reference range. The change in BCMP function or level due to the administration of such compounds can be readily detected, *e.g.*, by obtaining a sample (*e.g.,* a sample of breast tissue, blood or urine or a tissue sample such as biopsy tissue) and assaying *in vitro* the levels or activities of said BCMPs, or the levels of mRNAs encoding said BCMPs, or any combination of the foregoing. Such assays can be performed before and after the administration of the compound as described herein.

The compounds include but are not limited to any compound, *e.g.,* a small organic molecule, protein, peptide, antibody, nucleic acid, etc. that restores the BCMP profile towards normal with the proviso that such compounds or treatments does not include taxol, cyclophosphamide, tamoxifen, and doxorubacin. The compounds may be given in combination with any other compound, including taxol, cyclophosphamide, tamoxifen, and doxorubacin.

### Vaccine Therapy

BCMPs may be useful as antigenic material, and may be used in the production of vaccines for treatment or prophylaxis of breast cancer. Such material can be "antigenic" and/or "immunogenic". Generally, "antigenic" is taken to mean that the protein is capable of being used to raise antibodies or indeed is capable of inducing an antibody response in a subject. "Immunogenic" is taken to mean that the protein is capable of eliciting a protective immune response in a subject. Thus, in the latter case, the protein may be capable of not only generating an antibody response but, in addition, non-antibody based immune responses.

The skilled person will appreciate that homologues or derivatives of the BCMPs will also find use as antigenic/immunogenic material. Thus, for instance proteins which include one or more additions, deletions, substitutions or the like are useful in the present invention. In addition, it may be possible to replace one amino acid with another of similar "type". For instance, replacing one hydrophobic amino acid with another. One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated.

In the case of homologues and derivatives, the degree of identity with a protein as described herein is less important than that the homologue or derivative should retain its antigenicity and/or immunogenicity. However, suitably, homologues or derivatives having at least 60% similarity (as discussed above) with the proteins or polypeptides described herein are provided. Preferably, homologues or derivatives having at least 70% similarity, more preferably at least 80% similarity are provided. Most preferably, homologues or derivatives having at least 90% or even 95% similarity are provided.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient antigenicity to be useful.

It is well known that it is possible to screen an antigenic protein or polypeptide to identify epitopic regions, i.e. those regions which are responsible for the protein or polypeptide's antigenicity or immunogenicity. Methods well known to the skilled person can be used to test fragments and/or homologues and/or derivatives for antigenicity. Thus, the fragments should include one or more such epitopic regions or be sufficiently similar to such regions to retain their antigenic/immunogenic properties. Thus, for fragments useful according to the present invention the degree of identity is perhaps irrelevant, since they may be 100% identical to a particular part of a protein or polypeptide, homologue or derivative as described herein. The key issue, once again, is that the fragment retains the antigenic/immunogenic properties of the protein from which it is derived.

What is important for homologues, derivatives and fragments is that they possess at least a degree of the antigenicity/immunogenicity of the protein or polypeptide from which they are derived. Thus, in an additional aspect of the invention, there is provided the use of antigenic/or immunogenic fragments of a BCMP, or of homologues or derivatives thereof.

The BCMPs, or antigenic fragments thereof, can be provided alone, as a purified or isolated preparation. They may be provided as part of a mixture with one or more other proteins, or antigenic fragments thereof. In a further aspect, therefore, the invention provides the use of an antigen composition comprising one or more BCMPs and/or one or more antigenic fragments thereof. Such a composition can be used for the detection and/or diagnosis of breast cancer.

In a sixth aspect, the present invention provides a method of detecting and/or diagnosing breast cancer which comprises:
bringing into contact with a sample to be tested an antigenic BCMP, or an antigenic fragment thereof, or an antigen composition; and
detecting the presence of antibodies to breast cancer.

In particular, the protein, antigenic fragment thereof or antigen composition can be used to detect IgA, IgM or IgG antibodies. Suitably, the sample to be tested will be a biological sample, e.g. a sample of blood or saliva.

In a further aspect, the invention provides the use of an antigenic BCMP, antigenic fragment thereof or an antigenic composition in detecting and/or diagnosing breast cancer. Preferably, the detecting and/or diagnosing is carried out *in vitro.*

The antigenic BCMPs, antigenic fragments thereof or antigenic composition useful in the present invention can be provided as a kit for use in the *in vitro* detection and/or diagnosis of breast cancer. Thus, in a still further aspect, the present invention provides the use of a kit for use in the detection and/or diagnosis of breast cancer, which kit comprises an antigenic BCMP, an antigenic fragment thereof or an antigenic composition.

In addition, the antigenic BCMP, antigenic fragment thereof or antigen composition can be used to induce an immune response against breast cancer. Thus, in a yet further aspect, the invention provides the use of an antigenic BCMP, an antigenic fragment thereof or an antigen composition in medicine.

In a further aspect, the present invention provides the use of a composition capable of eliciting an immune response in a subject, which composition comprises a BCMP, an antigenic fragment thereof, or an antigen composition of the invention. Suitably, the composition will be a vaccine composition, optionally comprising one or more suitable adjuvants. Such a vaccine composition may be either a prophylactic or therapeutic vaccine composition.

The vaccine compositions useful in the invention can include one or more adjuvants. Examples well-known in the art include inorganic gels, such as aluminium hydroxide, and water-in-oil emulsions, such as incomplete Freund's adjuvant. Other useful adjuvants will be well known to the skilled person.

In yet further aspects, the present invention provides the use of a BCMP, an antigenic fragment thereof, or an antigen composition in the preparation of an immunogenic composition, preferably a vaccine.

Such an immunogenic composition is useful for inducing an immune response in a subject; and can be used in a method for the treatment or prophylaxis of breast cancer in a subject, or of vaccinating a subject against breast cancer which comprises the step of administering to the subject an effective amount of a BCMP, at least one antigenic fragment thereof or an antigen composition, preferably as a vaccine.

In a specific embodiment, a preparation ofBCMPs or BCMP peptide fragments defined above is used as a vaccine for the treatment of breast cancer. Such preparations may include adjuvants or other vehicles.

In another embodiment, a preparation of oligonucleotides comprising 10 or more consecutive nucleotides complementary to a nucleotide sequence encoding BCMPs or BCMP peptide fragments defined above for use as vaccines for the treatment of breast cancer. Such preparations may include adjuvants or other vehicles.

### Gene Therapy

In a specific embodiment, nucleic acids comprising a sequence encoding a BCMP, a BCMP fragment, BCMP-related polypeptide or fragment of a BCMP-related polypeptide, are administered to promote BCMP function by way of gene therapy. Gene therapy refers to administration to a subject of an expressed or expressible nucleic acid. In this embodiment, the nucleic acid produces its encoded polypeptide that mediates a therapeutic effect by promoting BCMP function.

Any of the methods for gene therapy available in the art can be used according to the present invention. Exemplary methods are described below.

For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62:191-217; May, 1993, TIBTECH 11(5):155-215. Methods commonly known in the art of recombinant DNA technology which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

In a preferred aspect, the compound comprises a nucleic acid encoding a BCMP or fragment or chimeric protein thereof, said nucleic acid being part of an expression vector that expresses a BCMP or fragment or chimeric protein thereof in a suitable host. In particular, such a nucleic acid has a promoter operably linked to the BCMP coding region, said promoter being inducible or constitutive (and, optionally, tissue-specific). In another particular embodiment, a nucleic acid molecule is used in which the BCMP coding sequences and any other desired sequences are flanked by regions that promote homologous recombination at a desired site in the genome, thus providing for intrachromosomal expression of the BCMP nucleic acid (Koller and Smithies, 1989, Proc. Natl. Acad. Sci. USA 86:8932-8935; Zijlstra et al., 1989, Nature 342:435-438).

Delivery of the nucleic acid into a subject may be direct, in which case the subject is directly exposed to the nucleic acid or nucleic acid-carrying vector; this approach is known as *in vivo* gene therapy. Alternatively, delivery of the nucleic acid into the subject may be indirect, in which case cells are first transformed with the nucleic acid *in vitro* and then transplanted into the subject; this approach is known as *ex vivo* gene therapy.

In a specific embodiment, the nucleic acid is directly administered *in vivo,* where it is expressed to produce the encoded product. This can be accomplished by any of numerous methods known in the art, *e.g.,* by constructing it as part of an appropriate nucleic acid expression vector and administering it so that it becomes intracellular, *e.g.,* by infection using a defective or attenuated retroviral or other viral vector (see U.S. Patent No. 4,980,286); by direct injection of naked DNA; by use of microparticle bombardment (*e.g.*, a gene gun; Biolistic, Dupont); by coating with lipids, cell-surface receptors or transfecting agents; by encapsulation in liposomes, microparticles or microcapsules; by administering it in linkage to a peptide which is known to enter the nucleus; or by administering it in linkage to a ligand subject to receptor-mediated endocytosis (see*, e.g.,* Wu and Wu, 1987, J. Biol. Chem. 262:4429-4432), which can be used to target cell types specifically expressing the receptors. In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (see, *e.g.,* PCT Publications WO 92/06180 dated April 16, 1992 (Wu et al.); WO 92/22635 dated December 23, 1992 (Wilson et al.); WO92/20316 dated November 26, 1992 (Findeis et al.); WO93/14188 dated July 22, 1993 (Clarke et al.), WO 93/20221 dated October 14, 1993 (Young)). Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

In a specific embodiment, a viral vector that contains a nucleic acid encoding a BCMP is used. For example, a retroviral vector can be used (see Miller et al., 1993, Meth. Enzymol. 217:581-599). These retroviral vectors have been modified to delete retroviral sequences that are not necessary for packaging of the viral genome and integration into host cell DNA. The nucleic acid encoding the BCMP to be used in gene therapy is cloned into the vector, which facilitates delivery of the gene into a subject. More detail about retroviral vectors can be found in Boesen et al., 1994, Biotherapy 6:291-302, which describes the use of a retroviral vector to deliver the mdrl gene to hematopoietic stem cells in order to make the stem cells more resistant to chemotherapy. Other references illustrating the use of retroviral vectors in gene therapy are: Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, 1993, Current Opinion in Genetics and Development 3:499-503 present a review of adenovirus-based gene therapy. Bout et al., 1994, Human Gene Therapy 5:3-10 demonstrated the use of adenovirus vectors to transfer genes to the respiratory epithelia of rhesus monkeys. Other instances of the use of adenoviruses in gene therapy can be found in Rosenfeld et al., 1991, Science 252:431-434; Rosenfeld et al., 1992, Cell 68:143-155; Mastrangeli et al., 1993, J. Clin. Invest. 91:225-234; PCT Publication WO94/12649; and Wang, et al., 1995, Gene Therapy 2:775-783.

Adeno-associated virus (AAV) has also been proposed for use in gene therapy (Walsh et al., 1993, Proc. Soc. Exp. Biol. Med. 204:289-300; U.S. Patent No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a subject.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, *e.g.,* Loeffler and Behr, 1993, Meth. Enzymol. 217:599-618; Cohen et al., 1993, Meth. Enzymol. 217:618-644; Cline, 1985, Pharmac. Ther. 29:69-92) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a subject by various methods known in the art. In a preferred embodiment, epithelial cells are injected, *e.g.,* subcutaneously. In another embodiment, recombinant skin cells may be applied as a skin graft onto the subject. Recombinant blood cells *(e.g.,* hematopoietic stem or progenitor cells) are preferably administered intravenously. The amount of cells envisioned for use depends on the desired effect, the condition of the subject, etc., and can be determined by one skilled in the art.

Cells into which a nucleic acid can be introduced for purposes of gene therapy encompass any desired, available cell type, and include but are not limited to neuronal cells, glial cells (e.g., oligodendrocytes or astrocytes), epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood or fetal liver.

In a preferred embodiment, the cell used for gene therapy is autologous to the subject that is treated.

In an embodiment in which recombinant cells are used in gene therapy, a nucleic acid encoding a BCMP is introduced into the cells such that it is expressible by the cells or their progeny, and the recombinant cells are then administered *in vivo* for therapeutic effect. In a specific embodiment, stem or progenitor cells are used. Any stem or progenitor cells which can be isolated and maintained *in vitro* can be used in accordance with this embodiment of the present invention (see e.g. PCT Publication WO 94/08598, dated April 28, 1994; Stemple and Anderson, 1992, Cell 71:973-985; Rheinwald, 1980, Meth. Cell Bio. 21A:229; and Pittelkow and Scott, 1986, Mayo Clinic Proc. 61:771).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

Direct-injection of a DNA coding for a BCMP may also be performed according to, for example, the techniques described in United States Patent No. 5,589,466. These techniques involve the injection of "naked DNA", *i.e.,* isolated DNA molecules in the absence of liposomes, cells, or any other material besides a suitable carrier. The injection of DNA encoding a protein and operably linked to a suitable promoter results in the production of the protein in cells near the site of injection and the elicitation of an immune response in the subject to the protein encoded by the injected DNA. In a preferred embodiment, naked DNA comprising (a) DNA encoding a BCMP and (b) a promoter are injected into a subject to elicit an immune response to the BCMP.

### Inhibition of BCMPs to Treat Breast Cancer

In one embodiment of the invention, breast cancer is treated or prevented by administration of a compound that antagonizes (inhibits) the level(s) and/or function(s) of one or more BCMPs which are elevated in the breast tissue of subjects having breast cancer as compared with breast tissue of subjects free from breast cancer.

Compounds useful for this purpose include but are not limited to anti-BCMP antibodies (and fragments and derivatives containing the binding region thereof), BCMP antisense or ribozyme nucleic acids, and nucleic acids encoding dysfunctional BCMPs that are used to "knockout" endogenous BCMP function by homologous recombination (see, *e.g.,* Capecchi, 1989, *Science* 244:1288-1292). Other compounds that inhibit BCMP function can be identified by use of known *in vitro* assays, *e.g.,* assays for the ability of a test compound to inhibit binding of a BCMP to another protein or a binding partner, or to inhibit a known BCMP function. Preferably such inhibition is assayed *in vitro* or in cell culture, but genetic assays may also be employed. The Preferred Technology can also be used to detect levels of the BCMPs before and after the administration of the compound. Preferably, suitable *in vitro* or *in vivo* assays are utilized to determine the effect of a specific compound and whether its administration is indicated for treatment of the affected tissue, as described in more detail below.

In a specific embodiment, a compound that inhibits a BCMP function is administered therapeutically or prophylactically to a subject in whom an increased breast tissue level or functional activity of the BCMP (*e.g.,* greater than the normal level or desired level) is detected as compared with breast tissue of subjects free from breast cancer or a predetermined reference range. Methods standard in the art can be employed to measure the increase in a BCMP level or function, as outlined above. Preferred BCMP inhibitor compositions include small molecules, i.e., molecules of 1000 daltons or less. Such small molecules can be identified by the screening methods described herein.

### Antisense Regulation of BCMPs

In a specific embodiment, BCMP expression is inhibited by use of BCMP antisense nucleic acids. The present invention provides the therapeutic or prophylactic use of nucleic acids comprising at least six nucleotides that are antisense to a gene or cDNA encoding a BCMP or a portion thereof. As used herein, a BCMP "antisense" nucleic acid refers to a nucleic acid capable of hybridizing by virtue of some sequence complementarity to a portion of an RNA (preferably mRNA) encoding a BCMP. The antisense nucleic acid may be complementary to a coding and/or noncoding region of an mRNA encoding a BCMP. Such antisense nucleic acids have utility as compounds that inhibit BCMP expression, and can be used in the treatment or prevention of breast cancer.

The antisense nucleic acids are double-stranded or single-stranded oligonucleotides, RNA or DNA or a modification or derivative thereof, and can be directly administered to a cell or produced intracellularly by transcription of exogenous, introduced sequences.

The invention can use pharmaceutical compositions comprising an effective amount of the BCMP antisense nucleic acids in a pharmaceutically acceptable carrier, as described *infra.*

In another embodiment, the invention is useful in methods for inhibiting the expression of a BCMP nucleic acid sequence in a prokaryotic or eukaryotic cell comprising providing the cell with an effective amount of a composition comprising a BCMP antisense nucleic acid of the invention.

BCMP antisense nucleic acids and their uses are described in detail below.

### BCMP Antisense Nucleic Acids

The BCMP antisense nucleic acids are of at least six nucleotides and are preferably oligonucleotides ranging from 6 to about 50 oligonucleotides. In specific aspects, the oligonucleotide is at least 10 nucleotides, at least 15 nucleotides, at least 100 nucleotides, or at least 200 nucleotides. The oligonucleotides can be DNA or RNA or chimeric mixtures or derivatives or modified versions thereof and can be single-stranded or double-stranded. The oligonucleotide can be modified at the base moiety, sugar moiety, or phosphate backbone. The oligonucleotide may include other appended groups such as peptides; agents that facilitate transport across the cell membrane (see, *e.g.,* Letsinger et al., 1989, Proc. Natl. Acad. Sci. USA 86:6553-6556; Lemaitre et al., 1987, Proc. Natl. Acad. Sci. 84:648-652; PCT Publication No. WO 88/09810, published December 15, 1988) or blood-brain barrier (see, *e.g.,* PCT Publication No. WO 89/10134, published April 25, 1988); hybridization-triggered cleavage agents (see, *e.g.,* Krol et al., 1988, BioTechniques 6:958-976) or intercalating agents (see, *e.g.,* Zon, 1988, Pharm. Res. 5:539-549).

In a preferred aspect, a BCMP antisense oligonucleotide is provided, preferably of single-stranded DNA. The oligonucleotide may be modified at any position on its structure with substituents generally known in the art.

The BCMP antisense oligonucleotide may comprise at least one of the following modified base moieties: 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, 2,6-diaminopurine, and other base analogs.

In another embodiment, the oligonucleotide comprises at least one modified sugar moiety, *e.g.,* one of the following sugar moieties: arabinose, 2-fluoroarabinose, xylulose, and hexose.

In yet another embodiment, the oligonucleotide comprises at least one of the following modified phosphate backbones: a phosphorothioate, a phosphorodithioate, a phosphoramidothioate, a phosphoramidate, a phosphordiamidate, a methylphosphonate, an alkyl phosphotriester, a formacetal, or an analog of formacetal.

In yet another embodiment, the oligonucleotide is an α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641).

The oligonucleotide may be conjugated to another molecule, *e.g.,* a peptide, hybridization triggered cross-linking agent, transport agent, or hybridization-triggered cleavage agent.

Oligonucleotides may be synthesized by standard methods known in the art, e.g., by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16:3209), and methylphosphonate oligonucleotides can be prepared by use of controlled pore glass polymer supports (Sarin et al., 1988, *Proc. Natl. Acad Sci. USA* 85:7448-7451).

In a specific embodiment, the BCMP antisense nucleic acid is produced intracellularly by transcription from an exogenous sequence. For example, a vector can be introduced *in vivo* such that it is taken up by a cell, within which cell the vector or a portion thereof is transcribed, producing an antisense nucleic acid (RNA). Such a vector would contain a sequence encoding the BCMP antisense nucleic acid. Such a vector can remain episomal or become chromosomally integrated, as long as it can be transcribed to produce the desired antisense RNA. Such vectors can be constructed by recombinant DNA technology standard in the art. Vectors can be plasmid, viral, or others known in the art, used for replication and expression in mammalian cells. Expression of the sequence encoding the BCMP antisense RNA can be by any promoter known in the art to act in mammalian, preferably human, cells. Such promoters can be inducible or constitutive. Examples of such promoters are outlined above.

The antisense nucleic acids comprise a sequence complementary to at least a portion of an RNA transcript of a gene encoding a BCMP, preferably a human gene encoding a BCMP. However, absolute complementarity, although preferred, is not required. A sequence "complementary to at least a portion of an RNA," as referred to herein, means a sequence having sufficient complementarity to be able to hybridize under stringent conditions (*e.g.,* highly stringent conditions comprising hybridization in 7% sodium dodecyl sulfate (SDS), 1 mM EDTA at 65 °C and washing in 0.1xSSC/0.1% SDS at 68 °C, or moderately stringent conditions comprising washing in 0.2xSSC/0.1% SDS at 42 °C) with the RNA, forming a stable duplex; in the case of double-stranded BCMP antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA encoding a BCMP it may contain and still form a stable duplex (or triplex, as the case may be). One skilled in the art can ascertain a tolerable degree of mismatch by use of standard procedures to determine the melting point of the hybridized complex.

### Therapeutic Use Of BCMP Antisense Nucleic Acids

The BCMP antisense nucleic acids can be used to treat or prevent breast cancer when the target BCMP is overexpressed in the breast tissue of subjects suspected of having or suffering from breast cancer. In a preferred embodiment, a single-stranded DNA antisense BCMP oligonucleotide is used.

Cell types which express or overexpress RNA encoding a BCMP can be identified by various methods known in the art. Such cell types include but are not limited to leukocytes (*e.g.,* neutrophils, macrophages, monocytes) and resident cells (*e.g*., astrocytes, glial cells, neuronal cells, and ependymal cells). Such methods include, but are not limited to, hybridization with a BCMP-specific nucleic acid (*e.g.,* by Northern hybridization, dot blot hybridization, *in situ* hybridization), observing the ability of RNA from the cell type to be translated *in vitro* into a BCMP, immunoassay, etc. In a preferred aspect, primary tissue from a subject can be assayed for BCMP expression prior to treatment, *e.g.,* by immunocytochemistry or *in situ* hybridization.

Pharmaceutical compositions, comprising an effective amount of a BCMP antisense nucleic acid in a pharmaceutically acceptable carrier, can be administered to a subject having breast cancer.

The amount of BCMP antisense nucleic acid which will be effective in the treatment of breast cancer can be determined by standard clinical techniques.

In a specific embodiment, pharmaceutical compositions comprising one or more BCMP antisense nucleic acids are administered via liposomes, microparticles, or microcapsules. In various embodiments, such compositions may be used to achieve sustained release of the BCMP antisense nucleic acids. In a specific embodiment, it may be desirable to use liposomes targeted via antibodies to specific identifiable tumor antigens (Leonetti et al., 1990, Proc. Natl. Acad. Sci. USA 87:2448-2451; Renneisen et al., 1990, J. Biol. Chem. 265:16337-16342).

### Inhibitory Ribozyme And Triple Helix Approaches

In another embodiment, symptoms of breast cancer may be ameliorated by decreasing the level of a BCMP or BCMP activity by using gene sequences encoding the BCMP in conjunction with well-known gene "knock-out," ribozyme or triple helix methods to decrease gene expression of a BCMP. In this approach ribozyme or triple helix molecules are used to modulate the activity, expression or synthesis of the gene encoding the BCMP, and thus to ameliorate the symptoms of breast cancer. Such molecules may be designed to reduce or inhibit expression of a mutant or non-mutant target gene. Techniques for the production and use of such molecules are well known to those of skill in the art.

Ribozyme molecules designed to catalytically cleave gene mRNA transcripts encoding a BCMP can be used to prevent translation of target gene mRNA and, therefore, expression of the gene product. (See, *e.g.,* PCT International Publication WO90/11364, published October 4, 1990; Sarver et al., 1990, Science 247:1222-1225).

Ribozymes are enzymatic RNA molecules capable of catalyzing the specific cleavage of RNA. (For a review, see Rossi, 1994, Current Biology 4, 469-471). The mechanism of ribozyme action involves sequence specific hybridization of the ribozyme molecule to complementary target RNA, followed by an endonucleolytic cleavage event. The composition of ribozyme molecules must include one or more sequences complementary to the target gene mRNA, and must include the well known catalytic sequence responsible for mRNA cleavage. For this sequence, see, e.g., U.S. Patent No. 5,093,246.

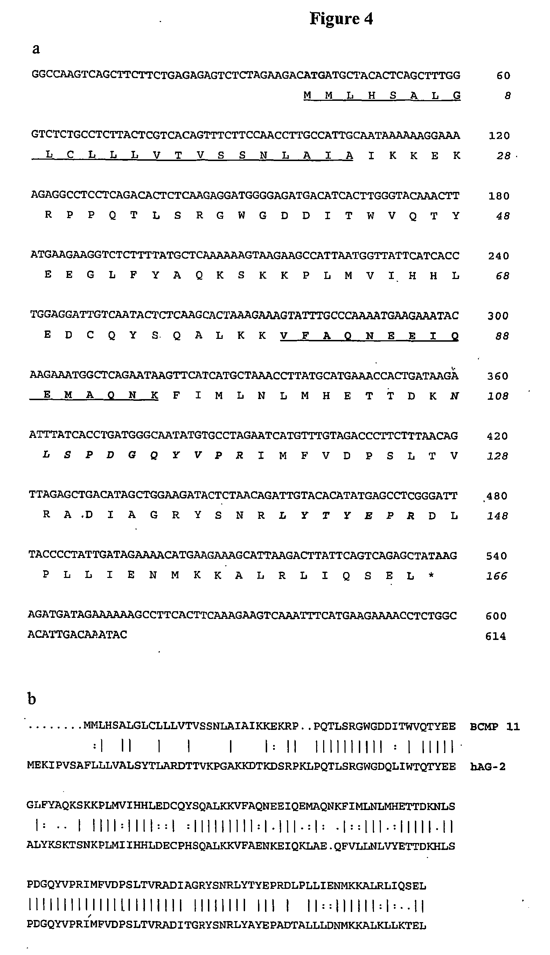

While ribozymes that cleave mRNA at site specific recognition sequences can be used to destroy mRNAs encoding a BCMP, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Myers, 1995, Molecular Biology and Biotechnology: A Comprehensive Desk Reference, VCH Publishers, New York, (see especially Figure 4, page 833) and in Haseloff and Gerlach, 1988, Nature, 334, 585-591.

Preferably the ribozyme is engineered so that the cleavage recognition site is located near the 5' end of the mRNA encoding the BCMP, *i.e.,* to increase efficiency and minimize the intracellular accumulation of non-functional mRNA transcripts.

The ribozymes also include RNA endoribonucleases (hereinafter "Cech-type ribozymes") such as the one that occurs naturally in *Tetrahymena thermophila* (known as the IVS, or L-19 IVS RNA) and that has been extensively described by Thomas Cech and collaborators (Zaug, et al., 1984, Science, 224, 574-578; Zaug and Cech, 1986, Science, 231, 470-475; Zaug, et al., 1986, Nature, 324, 429-433; published International patent application No. WO 88/04300 by University Patents Inc.; Been and Cech, 1986, Cell, 47, 207-216). The Cech-type ribozymes have an eight base pair active site which hybridizes to a target RNA sequence whereafter cleavage of the target RNA takes place. Cech-type ribozymes which target eight base-pair active site sequences that are present in the gene encoding the BCMP can be used according to the invention.

As in the antisense approach, the ribozymes can be composed of modified oligonucleotides *(e.g.,* for improved stability, targeting, etc.) and should be delivered to cells that express the BCMP *in vivo.* A preferred method of delivery involves using a DNA construct "encoding" the ribozyme under the control of a strong constitutive pol III or pol II promoter, so that transfected cells will produce sufficient quantities of the ribozyme to destroy endogenous mRNA encoding the BCMP and inhibit translation. Because ribozymes, unlike antisense molecules, are catalytic, a lower intracellular concentration is required for efficacy.

Endogenous BCMP expression can also be reduced by inactivating or "knocking out" the gene encoding the BCMP, or the promoter of such a gene, using targeted homologous recombination *(e.g.,* see Smithies, et al., 1985, Nature 317:230-234; Thomas and Capecchi, 1987, Cell 51:503-512; Thompson et al., 1989, Cell 5:313-321; and Zijlstra et al., 1989, Nature 342:435-438, each of which is incorporated by reference herein in its entirety). For example, a mutant gene encoding a non-functional BCMP (or a completely unrelated DNA sequence) flanked by DNA homologous to the endogenous gene (either the coding regions or regulatory regions of the gene encoding the BCMP) can be used, with or without a selectable marker and/or a negative selectable marker, to transfect cells that express the target gene *in vivo.* Insertion of the DNA construct, via targeted homologous recombination, results in inactivation of the target gene. Such approaches are particularly suited in the agricultural field where modifications to ES (embryonic stem) cells can be used to generate animal offspring with an inactive target gene (*e.g.,* see Thomas and Capecchi, 1987 and Thompson, 1989, *supra).* However this approach can be adapted for use in humans provided the recombinant DNA constructs are directly administered or targeted to the required site *in vivo* using appropriate viral vectors.

Alternatively, the endogenous expression of a gene encoding a BCMP can be reduced by targeting deoxyribonucleotide sequences complementary to the regulatory region of the gene (*i.e.,* the gene promoter and/or enhancers) to form triple helical structures that prevent transcription of the gene encoding the BCMP in target cells in the body. (See generally, Helene, 1991, Anticancer Drug Des., 6(6), 569-584; Helene, et al., 1992, Ann. N.Y. Acad. Sci., 660, 27-36; and Maher, 1992, Bioassays 14(12), 807-815).

Nucleic acid molecules to be used in triplex helix formation for the inhibition of transcription should be single stranded and composed of deoxynucleotides. The base composition of these oligonucleotides must be designed to promote triple helix formation via Hoogsteen base pairing rules, which generally require sizeable stretches of either purines or pyrimidines to be present on one strand of a duplex. Nucleotide sequences may be pyrimidine-based, which will result in TAT and CGC⁺ triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, contain a stretch of G residues. These molecules will form a triple helix with a DNA duplex that is rich in GC pairs, in which the majority of the purine residues are located on a single strand of the targeted duplex, resulting in GGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

In instances wherein the antisense, ribozyme, or triple helix molecules described herein are utilized to inhibit mutant gene expression, it is possible that the technique may so efficiently reduce or inhibit the transcription (triple helix) or translation (antisense, ribozyme) of mRNA produced by normal gene alleles of a BCMP that the situation may arise wherein the concentration of BCMP present may be lower than is necessary for a normal phenotype. In such cases, to ensure that substantially normal levels of activity of a gene encoding a BCMP are maintained, gene therapy may be used to introduce into cells nucleic acid molecules that encode and express the BCMP that exhibit normal gene activity and that do not contain sequences susceptible to whatever antisense, ribozyme, or triple helix treatments are being utilized. Alternatively, in instances whereby the gene encodes an extracellular protein, normal BCMPs can be co-administered in order to maintain the requisite level of BCMP activity.

Anti-sense RNA and DNA, ribozyme, and triple helix molecules may be prepared by any method known in the art for the synthesis of DNA and RNA molecules, as discussed above. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors that incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

### Assays for Therapeutic or Prophylactic Compounds

The present invention also provides assays for use in drug discovery in order to identify or verify the efficacy of compounds for treatment or prevention of breast cancer. Test compounds can be assayed for their ability to restore BCMP levels in a subject having breast cancer towards levels found in subjects free from breast cancer or to produce similar changes in experimental animal models of breast cancer. Compounds able to restore BCMP levels in a subject having breast cancer towards levels found in subjects free from breast cancer or to produce similar changes in experimental animal models of breast cancer can be used as lead compounds for further drug discovery, or used therapeutically. BCMP expression can be assayed by the Preferred Technology, immunoassays, gel electrophoresis followed by visualization, detection of BCMP activity, or any other method taught herein or known to those skilled in the art. Such assays can be used to screen candidate drugs, in clinical monitoring or in drug development, where abundance of a BCMP can serve as a surrogate marker for clinical disease.

In various specific embodiments, *in vitro* assays can be carried out with cells representative of cell types involved in a subject's disorder, to determine if a compound has a desired effect upon such cell types.

Compounds for use in therapy can be tested in suitable animal model systems prior to testing in humans, including but not limited to rats, mice, chicken, cows, monkeys, rabbits, etc. For *in vivo* testing, prior to administration to humans, any animal model system known in the art may be used. Examples of animal models of breast cancer include, but are not limited to xenografts of human breast cancer cell lines such as MDA-MB-435 in oestrogen-deprived Severe Combined Immunodeficient (SCID) mice (Eccles et al., 1994 Cell Biophysics 24/25, 279). These can be utilized to test compounds that modulate BCMP levels, since the pathology exhibited in these models is similar to that of breast cancer. It is also apparent to the skilled artisan that, based upon the present disclosure, transgenic animals can be produced with "knock-out" mutations of the gene or genes encoding one or more BCMPs. A "knock-out" mutation of a gene is a mutation that causes the mutated gene to not be expressed, or expressed in an aberrant form or at a low level, such that the activity associated with the gene product is nearly or entirely absent. Preferably, the transgenic animal is a mammal, more preferably, the transgenic animal is a mouse.

In one embodiment, test compounds that modulate the expression of a BCMP are identified in non-human animals (*e.g.,* mice, rats, monkeys, rabbits, and guinea pigs), preferably non-human animal models for breast cancer, expressing the BCMP. In accordance with this embodiment, a test compound or a control compound is administered to the animals, and the effect of the test compound on expression of one or more BCMPs is determined. A test compound that alters the expression of a BCMP (or a plurality of BCMPs) can be identified by comparing the level of the selected BCMP or BCMPs (or mRNA(s) encoding the same) in an animal or group of animals treated with a test compound with the level of the BCMP(s) or mRNA(s) in an animal or group of animals treated with a control compound. Techniques known to those of skill in the art can be used to determine the mRNA and protein levels, for example, *in situ* hybridization. The animals may or may not be sacrificed to assay the effects of a test compound.

In another embodiment, test compounds that modulate the activity of a BCMP or a biologically active portion thereof are identified in non-human animals *(e.g.,* mice, rats, monkeys, rabbits, and guinea pigs), preferably non-human animal models for breast cancer, expressing the BCMPs. In accordance with this embodiment, a test compound or a control compound is administered to the animals, and the effect of a test compound on the activity of a BCMP is determined. A test compound that alters the activity of a BCMP (or a plurality of BCMPs) can be identified by assaying animals treated with a control compound and animals treated with the test compound. The activity of the BCMP can be assessed by detecting induction of a cellular second messenger of the BCMP (*e.g*., intracellular Ca²⁺, diacylglycerol, IP3, etc.), detecting catalytic or enzymatic activity of the BCMP or binding partner thereof, detecting the induction of a reporter gene (*e.g.,* a regulatory element that is responsive to a BCMP operably linked to a nucleic acid encoding a detectable marker, such as luciferase or green fluorescent protein), or detecting a cellular response (*e.g.,* cellular differentiation or cell proliferation). Techniques known to those of skill in the art can be utilized to detect changes in the activity of a BCMP (see, *e.g.,* U.S. Patent No. 5,401,639, which is incorporated herein by reference).

In yet another embodiment, test compounds that modulate the level or expression of a BCMP (or plurality of BCMPs) are identified in human subjects having breast cancer, preferably those having severe breast cancer. In accordance with this embodiment, a test compound or a control compound is administered to the human subject, and the effect of a test compound on BCMP expression is determined by analyzing the expression of the BCMP or the mRNA encoding the same in a biological sample (*e.g*., breast tissue, serum, plasma, or urine). A test compound that alters the expression of a BCMP can be identified by comparing the level of the BCMP or mRNA encoding the same in a subject or group of subjects treated with a control compound to that in a subject or group of subjects treated with a test compound. Alternatively, alterations in the expression of a BCMP can be identified by comparing the level of the BCMP or mRNA encoding the same in a subject or group of subjects before and after the administration of a test compound. Techniques known to those of skill in the art can be used to obtain the biological sample and analyze the mRNA or protein expression. For example, the Preferred Technology described herein can be used to assess changes in the level of a BCMP.

In another embodiment, test compounds that modulate the activity of a BCMP (or plurality of BCMPs) are identified in human subjects having breast cancer, (preferably those with severe breast cancer). In this embodiment, a test compound or a control compound is administered to the human subject, and the effect of a test compound on the activity of a BCMP is determined. A test compound that alters the activity of a BCMP can be identified by comparing biological samples from subjects treated with a control compound to samples from subjects treated with the test compound. Alternatively, alterations in the activity of a BCMP can be identified by comparing the activity of a BCMP in a subject or group of subjects before and after the administration of a test compound. The activity of the BCMP can be assessed by detecting in a biological sample (*e.g*., breast tissue, serum, plasma, or urine) induction of a cellular signal transduction pathway of the BCMP *(e.g.,* intracellular Ca²⁺, diacylglycerol, IP3, etc.), catalytic or enzymatic activity of the BCMP or a binding partner thereof, or a cellular response, for example, cellular differentiation, or cell proliferation. Techniques known to those of skill in the art can be used to detect changes in the induction of a second messenger of a BCMP or changes in a cellular response. For example, RT-PCR can be used to detect changes in the induction of a cellular second messenger.

In a preferred embodiment, a test compound that changes the level or expression of a BCMP towards levels detected in control subjects (*e.g.,* humans free from breast cancer) is selected for further testing or therapeutic use. In another preferred embodiment, a test compound that changes the activity of a BCMP towards the activity found in control subjects (*e.g.,* humans free from breast cancer) is selected for further testing or therapeutic use.

In another embodiment, test compounds that reduce the severity of one or more symptoms associated with breast cancer are identified in human subjects having breast cancer, preferably subjects with severe breast cancer. In accordance with this embodiment, a test compound or a control compound is administered to the subjects, and the effect of a test compound on one or more symptoms of breast cancer is determined. A test compound that reduces one or more symptoms can be identified by comparing the subjects treated with a control compound to the subjects treated with the test compound. Techniques known to physicians familiar with breast cancer can be used to determine whether a test compound reduces one or more symptoms associated with breast cancer. For example, a test compound that reduces tumour burden in a subject having breast cancer will be beneficial for subjects having breast cancer.

In a preferred embodiment, a test compound that reduces the severity of one or more symptoms associated with breast cancer in a human having breast cancer is selected for further testing or therapeutic use.

### Therapeutic and Prophylactic Compositions and their Use

The invention is useful in methods of treatment (and prophylaxis) comprising administering to a subject an effective amount of an active compound. In a preferred aspect, the compound is substantially purified (*e.g.,* substantially free from substances that limit its effect or produce undesired side-effects). The subject is preferably an animal, including but not limited to animals such as cows, pigs, horses, chickens, cats, dogs, etc., and is preferably a mammal, and most preferably human. In a specific embodiment, a non-human mammal is the subject.

Formulations and methods of administration that can be employed when the compound comprises a nucleic acid are described above; additional appropriate formulations and routes of administration are described below.

Various delivery systems are known and can be used to administer the active compound, *e.g.*, encapsulation in liposomes, microparticles, microcapsules, recombinant cells capable of expressing the compound, receptor-mediated endocytosis (see, *e.g.,* Wu and Wu, 1987, J. Biol. Chem. 262:4429-4432), construction of a nucleic acid as part of a retroviral or other vector, etc. Methods of introduction can be enteral or parenteral and include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (*e.g.,* oral mucosa, rectal and intestinal mucosa, etc.) and may be administered together with other biologically active agents. Administration can be systemic or local. In addition, it may be desirable to introduce the pharmaceutical compositions into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g.*, by use of an inhaler or nebulizer, and formulation with an aerosolizing agent.

In a specific embodiment, it may be desirable to administer the pharmaceutical compositions locally to the area in need of treatment; this may be achieved, for example, and not by way of limitation, by local infusion during surgery, topical application, *e.g.,* by injection, by means of a catheter, or by means of an implant, said implant being of a porous, non-porous, or gelatinous material, including membranes, such as sialastic membranes, or fibers. In one embodiment, administration can be by direct injection into breast tissue or at the site (or former site) of a malignant tumor or neoplastic or pre-neoplastic tissue.

In another embodiment, the compound can be delivered in a vesicle, in particular a liposome (see Langer, 1990, Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, *ibid.*, pp. 317-327; *see* generally *ibid.*)

In yet another embodiment, the compound can be delivered in a controlled release system. In one embodiment, a pump may be used (see Langer, *supra*; Sefton, 1987, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., 1983, Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105). In yet another embodiment, a controlled release system can be placed in proximity of the therapeutic target, *i.e*., the breast, thus requiring only a fraction of the systemic dose (see, *e.g.,* Goodson, in Medical Applications of Controlled Release, *supra,* vol. 2, pp. 115-138 (1984)).

Other controlled release systems are discussed in the review by Langer (1990, Science 249:1527-1533).

In a specific embodiment where the compound is a nucleic acid encoding a protein, the nucleic acid can be administered *in vivo* to promote expression of its encoded protein, by constructing it as part of an appropriate nucleic acid expression vector and administering it so that it becomes intracellular, *e.g.,* by use of a retroviral vector (see U.S. Patent No. 4,980,286), or by direct injection, or by use of microparticle bombardment *(e.g.,* a gene gun; Biolistic, Dupont), or coating with lipids or cell-surface receptors or transfecting agents, or by administering it in linkage to a homeobox-like peptide which is known to enter the nucleus (see *e.g.,* Joliot et al., 1991, Proc. Natl. Acad. Sci. USA 88:1864-1868), etc. Alternatively, a nucleic acid can be introduced intracellularly and incorporated within host cell DNA for expression, by homologous recombination.

The present invention also uses pharmaceutical compositions. Such compositions comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the subject. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The active compounds can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the compound which will be effective in the treatment of breast cancer can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The invention also uses a pharmaceutical pack or kit comprising one or more containers filled with one or more of the ingredients of the pharmaceutical compositions. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

Examples 2, 3 and 4 are not intended to form part of the invention.

### EXAMPLE 1: IDENTIFICATION OF MEMBRANE PROTEINS EXPRESSED IN BREAST CANCER CELL LINES

Using the following Reference Protocol, proteins in breast cancer cell line membranes were separated by SDS-PAGE and analysed.

### MATERIALS AND METHODS

### 1a - Crude fractionation of adherent breast carcinoma cell lines

The human breast cell lines, MDA-MB-468 (ATCC:HTB-132), T47D (ATCC:HTB-133) and BT20 (ATCC:HTB-19) were cultured under various tissue culture conditions (For BT20 cell line: the culture medium was Modified Eagle's Medium, 10% Foetal calf serum, non-essential amino acid mixture, 2mM Glutamine, 1% penicillin + streptomycin. For T47D and MDA MB-468 cell lines the culture media was DMF12, 10% Foetal calf serum, 2mM Glutamine, 1% penicillin + streptomycin. The cells were cultured in the above culture media at 37°C in 5 % CO₂), The cells were lysed and fractionated according to the protocols described below.

5 ml of cold homogenation buffer (50 mM TrisHCl, 250 mM sucrose, 1mM EDTA pH 7.4) with anti-proteinase (Sigma Proteinase inhibitor cocktail P2714) was added to a series of ten 15 cm² culture dishes containing 10e8 breast carcinoma cells ( approx 10mg of protein). The cells were scraped off the plates in the cold homogenization buffer and transferred into a 5 ml bijou (performed on ice). The resulting sample was sonicated (on ice) using a MSE Soniprep 150 with a flat bottomed probe for 10 seconds at an amplitude of 5 microns. The samples were then decanted into a 15 ml falcon tube and centrifuged at 1000g for 5 minutes at 4°C. The resulting supernatant was transferred into 11 x 60 mm clear ultra-centrifugation tube - taking care not to disturb the pellet (insoluble protein), and the tube topped up with homogenization buffer to almost full. The tube was then centrifuged at 100 000 x g for 1 hour at 4°C and then placed on ice, and the supernatant (cytosol) removed.

The pellet (membrane fraction) was washed gently three times with ice cold PBS, and resuspended in wash buffer (50 mM TrisHCl, 1mM EDTA pH 7.4) containing anti-proteinase. It was then ground up gently in a glass homogeniser (5-10 strokes), transferred to a clean ultra-centrifuge tube and centrifuged at 100 000 x g for 1 hour at 4°C. This last step was then repeated, washing with 0.5 M NaCl to strip off the peripheral proteins. The washed membrane protein yield from 10e8 breast carcinoma cells was approx. 1-2 mg of protein.

### 1b - Extraction of washed membrane proteins with the detergent Tx114

This extraction provides three potential fractions: detergent insoluble, peripheral membrane protein and integral membrane protein

Crude cell membranes were prepared as described in 1a above and resuspended in 50mM Tris HCl, 1 mM EDTA 1.5% Triton X114, proteinase inhibitors, pH 7.4 via homogenisation using a Dounce homogeniser.

The Triton X114 (Tx114) membrane mixture was vortexed and incubated on ice for 30 min. Following this, the Tx114 membrane mixture was centrifuged for 10 min at 13000 g at 4°C and the supernatant carefully extracted from any detergent insoluble protein pellet.

The Tx114 supernatant was warmed to 37°C for 3 min in a water bath and then centrifuged at 13000 g for 3 min. The top aqueous layer was removed and the lower detergent phase resuspended in 1ml of Tris HCl, 0.2mM EDTA pH 7.4. This extraction was repeated two times further.

The aqueous phase represents hydrophilic membrane proteins and the detergent phase represents hydrophobic membrane protein.

The protein was extracted from the aqueous and detergent phases by chloroform-methanol extraction as described in 1d below. The yield of hydrophilic membrane proteins from 10e8 cells was approx. 0.5mg and the yield of hydrophobic membrane protein was approx. 0.1-0.2 mg. Finally, the membrane protein was solubilised in approx. 30 microlitres of 1D lysis buffer (1-2 µg/µl).

### 1c - Extraction of washed membrane proteins with the detergent digitonin

The washed membrane pellet from 1a was extracted using a detergent-containing buffer as follows.

Ice cold Digitonin buffer (0.01% in 50mM Tris HCl pH 7.4) was added to the membrane fraction, the resulting solution homogenised for 10 strokes and placed on ice for 30 minutes to allow protein to solubilise.

The sample was then centrifuged at 13000 x g for 5 minutes at 4°C to pellet any insoluble protein, and the supernatant extracted using the chloroform-methanol extraction as described in 1d below. The yield of digitonin extracted protein from 10e8 carcinoma cells was approx. 30-50 micrograms.

### 1d - Extraction of protein from aqueous or detergent solution using Chloroform/Methanol

400µl of methanol, 100µl of chloroform, and 300µl of water were added to a 100-200µl of a detergent solution from 1b and 1c. The resulting mixture was vortexed and shaken for 60 seconds and then centrifuged at 13000 x g for 10 minutes at 20°C.

Two layers were generated, with the required protein in the interface between the two layers. The top layer was carefully removed, 300µl of methanol added and the tube inverted, followed by centrifugation for 5 minutes at 13000g at 4°C to pellet the protein. The methanol was removed and the pellet allowed to dry in air for 5 to 10 minutes.

Following this the pellet was resolubilised 1D lysis buffer (63mM Tris.HCL pH7.4,10% Glycerol, 2% SDS, 0.0025% Bromophenol Blue, 2% Mercaptoethanol).

### 1e - 1D gel technology

Protein or membrane pellets were solubilised in 1D sample buffer (1-2 µg/µl). The sample buffer and protein mixture was then heated to 95°C for 3 min.

A 9-16% acrylamide gradient gel was cast with a stacking gel and a stacking comb according to the procedure described in Ausubel F.M. et al., eds., 1989, Current Protocols in Molecular Biology, Vol. II, Green Publishing Associates, Inc., and John Wiley & Sons, Inc., New York, section 10.2, incorporated herein by reference in its entirety.

30-50 micrograms of the protein mixtures obtained from detergent and the molecular weight standards (66, 45 ,31, 21,14 kDa) were added to the stacking gel wells using a 10 microlitre pipette tip and the samples run at 40mA for 5 hours.

The plates were then prised open, the gel placed in a tray of fixer (10% acetic acid, 40% ethanol, 50% water) and shaken overnight. Following this, the gel was primed by 30 minutes shaking in a primer solution (7.5% acetic acid (75mls), 0.05% SDS (5mls of 10%)). The gel was then incubated with a fluorescent dye (7.5% acetic acid, 0.06% OGS in-house dye (600µl) with shaking for 3 hrs. Sypro Red (Molecular Probes, Inc., Eugene, Oregon) is a suitable dye for this purpose. A preferred fluorescent dye is disclosed in U.S. Application No. 09/412,168, filed on October 5, 1999, which is incorporated herein by reference in its entirety.

A computer-readable output was produced by imaging the fluorescently stained gels with an Apollo 3 scanner (Oxford Glycosciences, Oxford, UK) This scanner is developed from the scanner described in WO 96/36882 and in the Ph.D. thesis of David A. Basiji, entitled "Development of a High-throughput Fluorescence Scanner Employing Internal Reflection Optics and Phase-sensitive Detection (Total Internal Reflection, Electrophoresis)", University of Washington (1997), Volume 58/12-B of Dissertation Abstracts International, page 6686, the contents of each of which are incorporated herein by reference. The latest embodiment of this instrument incudes the following improvements: The gel is transported through the scanner on a precision lead-screw drive system. This is preferrable to laying the glass plate on the belt-driven system that is defined in the Basiji thesis as it provides a reproducible means of accurately transporting the gel past the imaging optics.

The gel is secured into the scanner against three alignment stops that rigidly hold the glass plate in a known position. By doing this in conjunction with the above precision transport system and the fact that the gel is bound to the glass plate, the absolute position of the gel can be predicted and recorded. This ensures that accurate co-ordinates of each feature on the gel can be communicated to the cutting robot for excision. This cutting robot has an identical mounting arrangement for the glass plate to preserve the positional accuracy.

The carrier that holds the gel in place has integral fluorescent markers (Designated M1, M2, M3) that are used to correct the image geometry and are a quality control feature to confirm that the scanning has been performed correctly.

The optical components of the system have been inverted. The laser, mirror, waveguide and other optical components are now above the glass plate being scanned. The embodiment of the Basiji thesis has these underneath. The glass plate is therefore mounted onto the scanner gel side down, so that the optical path remains through the glass plate. By doing this, any particles of gel that may break away from the glass plate will fall onto the base of the instrument rather than into the optics.

In scanning the gels, they were removed from the stain, rinsed with water and allowed to air dry briefly and imaged on the Apollo 3. After imaging, the gels were sealed in polyethylene bags containing a small volume of staining solution, and then stored at 4°C.

Apparent molecular weights were calculated by interpolation from a set of known molecular weight markers run alongside the samples.

### 1f - Recovery and analysis of selected proteins

Proteins were robotically excised from the gels by the process described in U.S. Patent No. 6,064,754, Sections 5.4 and 5.6, 5.7, 5.8 (incorporated herein by reference),as is applicable to 1D-electrophoresis, with modification to the robotic cutter as follows: the cutter begins at the top of the lane, and cuts a gel disc 1.7mm in diameter from the left edge of the lane. the cutter then moves 2mm to the right, and 0.7mm down and cuts a further disc. This is then repeated. The cutter then moves back to a position directly underneath the first gel cut, but offset by 2.2mm downwards, and the pattern of three diagonal cuts are repeated. This is continued for the whole length of the gel.

NOTE: If the lane is observed to broaden significantly then a correction can be made also sideways i.e instead of returning to a position directly underneath a previous gel cut, the cut can be offset slightly to the left (on the left of the lane) and/or the right (on the right of the lane). The proteins contained within the gel fragments were processed to generate tryptic peptides; partial amino acid sequences of these peptides were determined by mass spectroscopy as described in WO98/53323 and Application No. 09/094,996, filed June 15, 1998.

Proteins were processed to generate tryptic digest peptides. Tryptic peptides were analyzed by mass spectrometry using a PerSeptive Biosystems Voyager- DETM STR Matrix-Assisted Laser Desorption Ionization Time-of-Flight (MALDI-TOF) mass spectrometer, and selected tryptic peptides were analyzed by tandem mass spectrometry (MS/MS) using a Micromass Quadrupole Time-of-Flight (Q-TOF) mass spectrometer (Micromass, Altrincham, U.K.) equipped with a nanoflowTM electrospray Z-spray source. For partial amino acid sequencing and identification of BCMPs uninterpreted tandem mass spectra of tryptic peptides were searched using the SEQUEST search program (Eng et al., 1994, J. Am. Soc. Mass Spectrom. 5:976-989), version v.C.1. Criteria for database identification included: the cleavage specificity of trypsin; the detection of a suite of a, b and y ions in peptides returned from the database, and a mass increment for all Cys residues to account for carbamidomethylation. The database searched was database constructed of protein entries in the non-redundant database held by the National Centre for Biotechnology Information (NCBI) which is accessible at http://www.ncbi.nlm.nih.gov/. Following identification of proteins through spectral-spectral correlation using the SEQUEST program, masses detected in MALDI-TOF mass spectra were assigned to tryptic digest peptides within the proteins identified. In cases where no amino acid sequences could be identified through searching with uninterpreted MS/MS spectra of tryptic digest peptides using the SEQUEST program, tandem mass spectra of the peptides were interpreted manually, using methods known in the art. (In the case of interpretation of low-energy fragmentation mass spectra of peptide ions see Gaskell et al., 1992, Rapid Commun. Mass Spectrom. 6:658-662)

### EXAMPLE 2: Mapping genes in genomic DNA sequences via mass spectrometry derived tandem spectra of trypsin digested protein fragments.

### Mass Spectrometry

For BCMP81, tryptic digests of proteins excised from the 1D gel were analysed by MALDI-TOF-MS (Voyager STR, Applied Biosystems, Framingham, MA) using a 337 nm wavelength laser for desorption and the reflectron mode of analysis. A selected mass ([M+H] =1557.81) was further characterised by tandem mass spectrometry using a QTOF-MS equipped with a nanospray ion source, (Micromass UK Ltd, Manchester). Prior to MALDI analysis the samples were desalted and concentrated using C18 Zip Tips^{™} (Millipore, Bedford, MA). Samples for tandem MS were purified using a nano LC system (LC Packings, Amsterdam, The Netherlands) incorporating C18 SPE material.

The tandem spectrum was analysed manually (Biemann K, Sequencing of peptides by Tandem Mass Spectrometry and high energy collision induced dissociation, Methods Enzymol 1990;193:455-79) to determine partial positional amino acid sequence along with the masses remaining to the N and C termini of the peptide fragments (Table 4).

**Table 4. Amino Acid Sequence Information Derived from Tandem Mass Spectrometry Analysis of BCMP 81**

| Peptide Precursor Ion m/z | Core Sequence^{a} | N-terminal mass^{b} | C-terminal mass^{c} |
|---|---|---|---|
| 1557.81 | MEQQQQLQQR | 241.2 | 0.00 |

| | | | |
|---|---|---|---|
| ^{a}The 'core sequence' is a partial amino acid sequence of a peptide eludicated from the interpretation of the fragment mass spectrum of the peptide. ^{b}The N-terminal mass of the peptide is the mass between the start of the core sequence and the N-terminus of the peptide. ^{c}The C-terminal mass is the mass between the end of the core sequence and the C-terminus of the peptide. | | | |

The sequence read from the tandem spectrum (MEQQQQLQQR) was found to be present in a translation of human genomic DNA from accession no. AL021707 (available at http://www.ncbi.nlm.nih.gov/entrez/) starting at nucleotide position 55443 (BLAST, Altschul, Stephen F., Gish, Warren, Miller, Webb, Myers, Eugene W., and Lipman, David J. (1990). Basic local alignment search tool. J. Mol. Biol. 215; 403-410. Gapped BLAST is described in Altschul, Stephen F., Madden, Thomas L., Schaffer, Alejandro A., Zhang, Jinghui, Zhang, Zheng, Miller, Webb, and Lipman, David J. (1997). GappedBLAST and PSI-BLAST: a new generation of protein database search programs. Nucleic Acids Res. 25(17); 3389-3402).

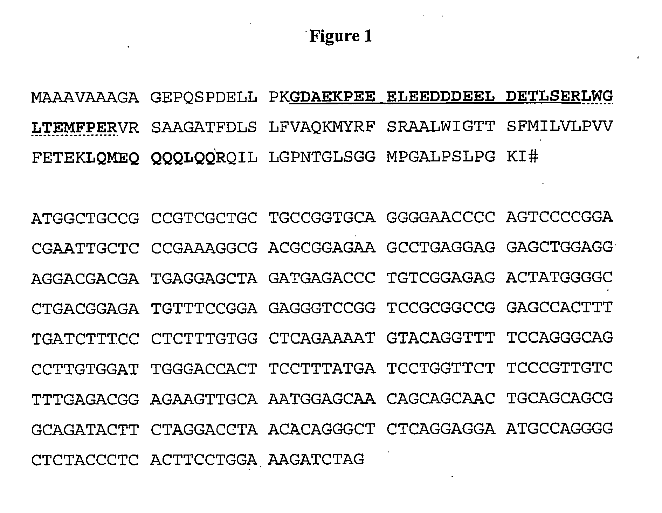

Further examination of sequences surrounding this position identifies DNA sequences that can be translated to match an additionnal MALDI mass spectrum. This mass spectrum was converted into a conceptual amino acid sequence (LWGLTEMFPER), the mass of which matches the potential trypsin fragment that can be translated from the DNA sequence (Figure 1). Thus, two sectors of this stretch of genomic DNA are defined in terms of reading frames e.g. the sequence derived from the tandem mass spectrum is followed closely by a stop codon in the translation of the genomic DNA, indicating either that this is the end of the gene or that there is an intron at this position. However, any potential intron must begin after the R residue at the C terminus of the tandem spectrum.

The genomic DNA sequence corresponding to 2kbs either side of the sequences matching the tandem spectrum were used in a blast search against Expressed Sequences Tags (ESTs) (http://www.ncbi.nlm.nih.gov). EST AA316462 matches at >99% against regions of this genomic sequence and assists identification of the intron and exon boundaries. This alignment identifies 4 exons but the EST sequences are not of sufficient quality to determine the protein sequence of the entire gene, nor are they good enough to precisely position the intron / exon boundaries, particularly in the absence of any homologous sequences. A final mass spectrum can be matched to a translation of the genomic sequence (GDAEKPEEELEEDDDEELDETLSER) once a conceptual splice is made from 54575 (DDEe(ag) / gt) to 54805 (e(ag) / LDET) in AL021707, the approximate positions of these splice sites having been determined from the ESTs.

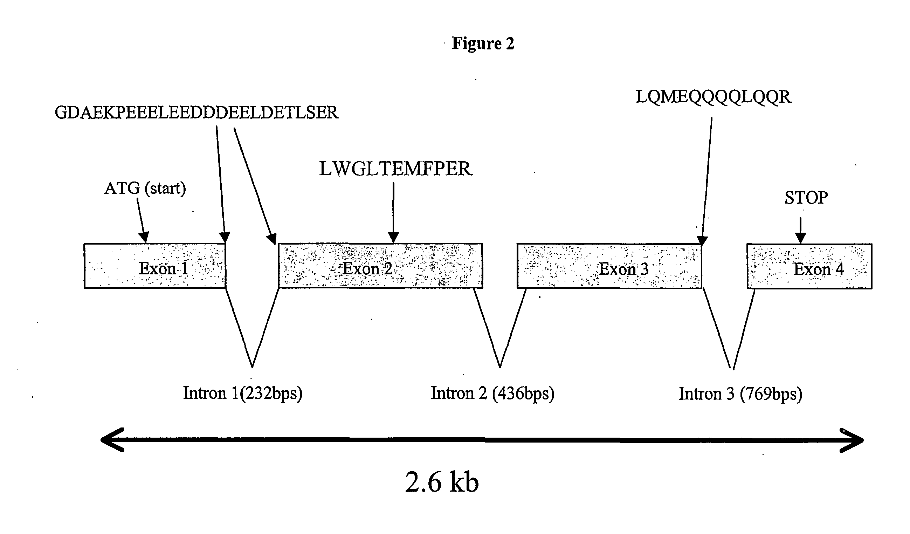

There is a CpG island starting at position 54283 in AL021707. CpG islands in human genomic DNA sequences often indicate regions where genes may begin (e.g. Kundu TK, Rao MR, CpG islands in chromatin organization and gene expression. J *Biochem (Tokyo)* 1999 Feb;125(2):217-22). An open reading frame is apparent from nucleotide 54461 of AL021707 beginning with ATG (methionine). The stop codon in exon 4 of this gene is defined by following the reading frame set by the spectra through the 3' EST sequences from AA316462. This combination of spectra, genomic DNA sequences and EST sequences yields the claimed protein sequence given below (Figure 1). The corresponding DNA sequence is given below (Figure 1). The positions of the spectra sequences, introns, and exons within the genomic fragment are given in Figure 2.
A full length clone was identified

### Total RNA preparation

Total RNA was prepared from ca. 10⁸ cells from cultures of cell lines MB-MDA468, BT20, PC3, PC3M, T47D, and Cal51 using Trizol (Life Technologies) and resuspended at ca. 1µg µl.

### cDNA cloning

The predicted coding region of BCMP 81 was amplified by PCR using primers BCMP 81 F (5'cctacagtcatggctgccgc3'), and BCMP 81 R (5'gagacagctcaaacagcaataac3'), 30ng mammary gland cDNA (Clontech), and PCR reagents (Qiagen, UK) using the cycling parameters : 40 cycles of 95°C for 15s, 55°C for 1 min. A single 500bp product was obtained, column purified from other PCR reagents (Qiagen, UK) and sequenced with primers BCMP 81 F and BCMP 81 R (ABI377, Oxford University Contract Sequencing)

The full length cDNA fragment that encodes BCMP 81 has been cloned from a mammary gland cDNA library and sequenced to confirm identity and reading frame. The molecular weight of the protein as cut from the 1D gel was between 14 and 19kDa, determined from a protein mass ladder, the predicted mass of the conceptually translated full length protein is 15.3kDa.

Although a CpG island is readily identified and noted in the GenBank annotation in the region of the peptide / genomic DNA match, no protein is predicted from these DNA sequences. The peptide data have enabled a full description of the protein, the mRNA that is translated, and the genomic structure of the gene that encodes it.

### mRNA profile of BCMP 81 in normal human tissue and breast and prostate cancer cell lines

Real time RT-PCR was used to quantitatively measure BCMP 81 expression in normal human tissue mRNAs (Clontech) and breast cancer cell lines. cDNAs were synthesized from 1-5µg of total RNA using an oligo dT primer and the Superscript II RT kit (Life technologies). Reactions containing 10ng cDNA, 10pmoles of sense (5'gagctagatgagaccctgtc3') and antisense (5'gatcataaaggaagtcccaa3') primers and SYBR green sequence detection reagents (PE Biosystems, UK) were assayed on an ABI7700 sequence detection system (PE Biosystems, UK). The PCR conditions were 1 cycle at 50°C for 2 min, 1 cycle at 95°C for 10 min, followed by 40 cycles of 95°C for 15s, 55°C for 1 min. The accumulation of PCR product was measured in real time as the increase in reporter dye fluorescence, and the data were analysed using the Sequence Detector program v1.6.3 (PE Biosystems). Standard curves relating initial template copy number to fluorescence and amplification cycle number were generated using the amplified PCR product as a template, and were used to calculate BCMP 81 mRNA copy number in each sample.

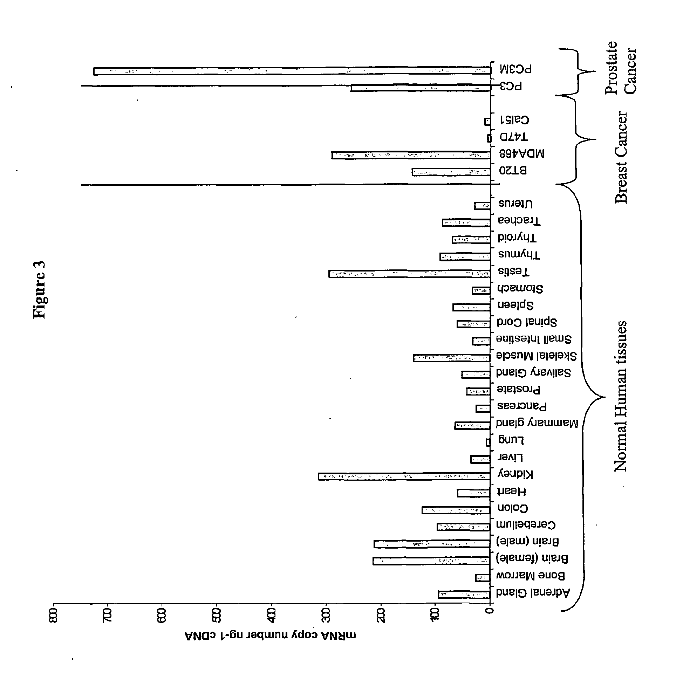

Analysis of the mRNA tissue distribution by quantitative RT-PCR (Heid, C.A., Stevens, J., Livak, K.J. & Williams, P.M. Real time quantitative PCR. *Genome Res.* **6,** 986-994 (1996)) verifies the source of the protein (MDA-MB468) and its potential as a breast cancer antigen with both BT20 and MDA-MB468 cell lines showing significantly increased expression over normal mammary gland (figure 3). The paucity of public database EST versions of this protein is reflected in the generally low mRNA copies (generally <200 per ng of cDNA) seen in these samples.

### EXAMPLE 3: CLONING OF THE NOVEL PROTEIN BCMP 11.

### Mass Spectrometry

Proteins excised from the 1D gel were digested with trypsin and analysed by MALDI-TOF-MS (Voyager STR, Applied Biosystems) using a 337 nm wavelength laser for desorption and the reflectron mode of analysis. Two selected mass for BCMP 11 ([M+H] = 1245.65 and 941.47) were further characterised by tandem mass spectrometry using a QTOF-MS equipped with a nanospray ion source, (Micromass UK Ltd.). Prior to MALDI analysis the samples were desalted and concentrated using C18 Zip TipsTM (Millipore). Samples for tandem MS were purified using a nano LC system (LC Packings) incorporating C18 SPE material.

The tandem spectra were analysed manually (Biemann K, Sequencing of peptides by Tandem Mass Spectrometry and high energy collision induced dissociation, Methods Enzymol 1990;193:455-79) to determine partial positional amino acid sequence along with the masses remaining to the N and C termini of the peptide fragments (Table 5).

Three spectra from protein BCMP 11 (one tandem spectrum, Table 5, and one MALDI-mass spectrum, Fig 4a), were found to match a translation of an EST from a human lung carcinoma library (accession number AI458391), defining an open reading frame (ORF) of 166 amino acids (Fig. 4a). A Blast search of a human EST database (http://www.ncbi.nlm.nih.gov/blast) with entry AI458391 identified several overlapping ESTs, providing additional 5' and 3' UTR sequences. A second peptide tandem spectrum from BCMP 11 (Table 5) identified a discrepancy in these EST sequences : the predicted codon 146 coded for either glutamine or arginine depending which EST was being translated. The tandem spectrum shows clearly that the arginine is present in the isolated protein; the glutamine being either a sequencing error or polymorphism.

**Table 5. Amino Acid Sequence Information Derived from Tandem Mass Spectrometry Analysis of BCMP 11**

| Peptide Precursor Ion m/z | Core Sequence^{a} | N-terminal mass^{b} | C-terminal mass^{c} |
|---|---|---|---|
| 1245.65 | SPDGQYVPR | 227.14 | 0.00 |
| 941.47 | LYTYEPR | 0.00 | 0.00 |

| | | | |
|---|---|---|---|
| ^{a}The 'core sequence' is a partial amino acid sequence of a peptide eludicated from the interpretation of the fragment mass spectrum of the peptide. ^{b}The N-terminal mass of the peptide is the mass between the start of the core sequence and the N-terminus of the peptide. ^{c}The C-terminal mass is the mass between the end of the core sequence and the C-terminus of the peptide. | | | |

A full length clone was amplified by PCR from T-47D cDNA (FIG 4A).

### Preparation of total RNA and cDNA synthesis

Total RNA was prepared from cultured cells and tissue samples using Trizol reagent (Life Technologies), according to the manufacturer's instructions, and resuspended in RNAse-free water at a concentration of 1µg/µl. 1 to 5µg total RNA were used as a template for cDNA synthesis using an oligo dT primer and the Superscript II reverse transcription kit (Life Technologies). cDNAs were column purified (Qiagen) and eluted at a concentration of 10ng/µl.

### Cloning of BCMP 11 cDNA

The predicted full length BCMP 11 ORF was amplified by PCR from T-47D cDNAs, using the following primers: F, 5' GGCCAAGTCAGCTTCTTCTG 3'; R, 5' GTATTTGTCAATGTGCCAGAGG 3'. Reactions contained 10ng cDNA and reagents for PCR (Qiagen), and used the following cycling parameters: 40 cycles of 94°C for 30 seconds, 60°C for 30 seconds. The PCR products were column purified (Qiagen), cloned into a T/A vector (Invitrogen) and the nucleotide sequence subsequently verified (University of Oxford, Sequencing Facility, UK).

The predicted BCMP 11 protein is highly homologous to hAG-2, a novel human protein encoded by a cDNA cloned from the MCF-7 breast cancer cell line (Thompson, D.A. & Weigel, R.J. hAG-2, the human homologue of the Xenopus laevis cement gland gene XAG-2, is co-expressed with estrogen receptor in breast cancer cell lines. *Biochem. Biophys. Res. Commun.* **251,** 111-116 (1998)). hAG-2 is the human homologue of XAG-2, a *Xenopus laevis* protein that is expressed in the cement gland during frog development (Aberger, F., Weidinger, G., Grunz, H. & Richter, K. Anterior specification of embryonic ectoderm: the role of the Xenopus cement gland-specific gene XAG-2. *Mech. Dev.* **72**, 115-130 (1998)). hAG-2 and BCMP 11 proteins are 71% identical, with relatively low sequence similarity at the N-terminii (Fig 4b). BCMP 11, like hAG-2, is predicted to be an extracellular protein with an N-tenninal signal sequence (http://psort.nibb.ac.jp).

### Expression of BCMP 11 mRNA in human tissues

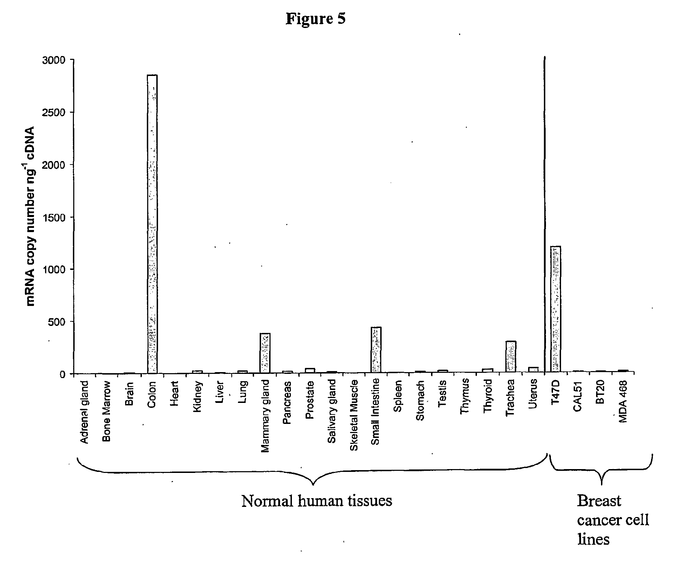

We used real time quantitative RT-PCR (Heid, C.A., Stevens, J., Livak, K.J. & Williams, P.M. Real time quantitative PCR. Genome Res. 6, 986-994 (1996); Morrison, T.B., Weis, J.J. & Wittwer, C.T. Quantification of low-copy transcripts by continuous SYBR Green I monitoring during amplification. Biotechniques 24, 954-958 (1998)) to analyse the distribution of BCMP 11 mRNA in normal human tissues and breast cancer cell lines (Fig 5). The primers used for PCR were as follows: sense, 5' CTGGAGGATTGTCAATACTC 3', antisense, 5' GCATAAGGTTTAGCATGATG 3'. Reactions containing 10ng cDNA, prepared as described above, SYBR green sequence detection reagents (PE Biosystems) and sense and antisense primers were assayed on an ABI7700 sequence detection system (PE Biosystems). The PCR conditions were 1 cycle at 50°C for 2 min, 1 cycle at 95°C for 10 min, and 40 cycles of 95°C for 15s, 55°C for 1min. The accumulation of PCR product was measured in real time as the increase in SYBR green fluorescence, and the data were analysed using the Sequence Detector program v1.6.3 (PE Biosystems). Standard curves relating initial template copy number to fluorescence and amplification cycle were generated using the amplified PCR product as a template, and were used to calculate BCMP 11 copy number in each sample.

The highest levels of BCMP 11 expression were observed in colon (2800 copies ng-1 cDNA), with lower levels of expression in mammary gland, small intestine and trachea (295-435 copies ng⁻¹ cDNA) (Fig. 5). BCMP 11 mRNA was also detected in T-47D cells (1200 copies ng⁻¹ cDNA), the source of BCMP 11 protein used in this study, and expression in this cell line was elevated in comparison to normal mammary tissue. Little or no BCMP 11 mRNA was detected in the other cell lines or normal tissues examined.

The tissue distribution of BCMP 11 and hAG-2 mRNAs is very similar. Both genes show a restricted pattern of expression in normal human tissues, with the colon being the major site of expression for both genes (Fig 5, Thompson, D.A. & Weigel, R.J. hAG-2, the human homologue of the Xenopus laevis cement gland gene XAG-2, is co-expressed with estrogen receptor in breast cancer cell lines. *Biochem. Biophys. Res. Commun.* **251**, 111-116 (1998)). hAG-2 expression was shown to be coincident with expression of the ER, and hAG-2 mRNA levels in MCF7 cells increased when the cells were treated with estradiol (Thompson and Weigel, *supra).* Similarly, BCMP 11 mRNA was detected in ER positive T-47D cells, but no expression was observed in the ER negative cell lines CAL51, BT20 and MDA-MB-468 (Figure 5). The coincident upregulation of two highly homologous genes that reside next to each other on chromosome 7 (see Chromosomal localisation, below) suggests a mechanism for coordinated control that may be linked not only to ER status but also to ER directed therapies such as Tamoxifen.

### Chromosomal localisation

A Blast search of a human genomic database with the BCMP 11 cDNA sequence (Fig. 4a) identified two entries, AC004993 and AC073333, with identity to the 5' and 3' ends of BCMP 11, respectively. Both clones have been mapped to chromosome 7p21. The entire hAG-2 ORF can also be mapped to the same contig as BCMP 11 in entry AC073333, demonstrating that these highly homologous genes are clustered together in the human genome.

### EXAMPLE 4: CLONING OF THE NOVEL PROTEIN BCMP 84.

### Mass Spectrometry

Proteins excised from the 1D gel were digested,with trypsin and analysed by MALDI-TOF-MS (Voyager STR, Applied Biosystems) using a 337 nm wavelength laser for desorption and the reflectron mode of analysis. A selected mass for BCMP 84 ([M+H] =1667.73) was further characterised by tandem mass spectrometry using a QTOF-MS equipped with a nanospray ion source, (Micromass UK Ltd.). Prior to MALDI analysis the samples were desalted and concentrated using C18 Zip Tips^{™} (Millipore). Samples for tandem MS were purified using a nano LC system (LC Packings) incorporating C18 SPE material.

The tandem spectrum was analysed manually (Biemann K, Sequencing of peptides by Tandem Mass Spectrometry and high energy collision induced dissociation, Methods Enzymol 1990;193:455-79) to determine partial positional amino acid sequence along with the masses remaining to the N and C termini of the peptide fragments (Table 6).

**Table 6. Amino Acid Sequence Information Derived from Tandem Mass Spectrometry Analysis of BCMP 84**

| Peptide Precursor Ion m/z | Core Sequence^{a} | N-terminal mass^{b} | C-terminal mass^{c} |
|---|---|---|---|
| 1667.73 | AEDAQEFSDVER | 272.19 | 0.00 |

| | | | |
|---|---|---|---|
| ^{a}The 'core sequence' is a partial amino acid sequence of a peptide eludicated from the interpretation of the fragment mass spectrum of the peptide. ^{b}The N-terminal mass of the peptide is the mass between the start of the core sequence and the N-terminus of the peptide. ^{c}The C-terminal mass is the mass between the end of the core sequence and the C-terminus of the peptide. | | | |

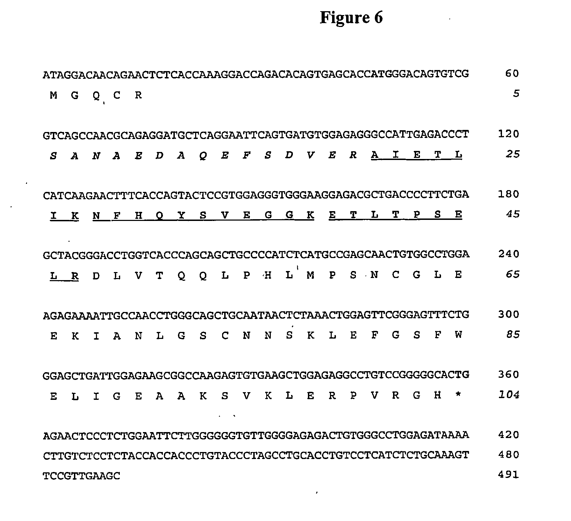

The tandem amino acid sequence and three MALDI-mass spectra were found to match a translation of an EST from a human colon carcinoma cell line (accession number AA315020) (Fig 6). Overlapping ESTs were identified which established a complete ORF of 104 amino acids.

A full length clone was amplified by PCR from MDA-MB-468 cDNA.

### Preparation of total RNA and cDNA synthesis

Total RNA was prepared from cultured cells and tissue samples using Trizol reagent (Life Technologies), according to the manufacturer's instructions, and resuspended in RNAse-free water at a concentration of 1µg/µl. 1 to 5µg total RNA were used as a template for cDNA synthesis using an oligo dT primer and the Superscript II reverse transcription kit (Life Technologies). cDNAs were column purified (Qiagen) and eluted at a concentration of 10ng/µl.

### Cloning of BCMP 84 cDNA

The predicted full length BCMP 84 ORF was amplified by PCR from MDA-MB-468 cDNAs, using the following primers: F, 5' ATAGGACAACAGAACTCTCACC 3'; R, 5' GCTTCAACGGAACTTTGCAGAG 3'. Reactions contained 10ng cDNA and reagents for PCR (Qiagen), and used the following cycling parameters: 40 cycles of 94°C for 30 seconds, 60°C for 30 seconds. The PCR products were column purified (Qiagen), cloned into a T/A vector (Invitrogen) and the nucleotide sequence subsequently verified (University of Oxford, Sequencing Facility, UK).

The predicted BCMP 84 protein shows similarity to the S100 family of calcium binding proteins (eg. S100A13, accession number Q99584 has 36% identity and 67% homology with BCMP 84) and a recently identified cDNA (AAG01893), which is identical to BCMP 84 over most of its length, has been named S 1 00A14 and annotated as a novel member of the S 100 family of calcium binding proteins. However, there has been no demonstration that AAG01893/S100A14 binds calcium and this gene and BCMP 84 lack the calcium binding motifs that are conserved between members of this protein family. The 2 amino acid differences between BCMP 84 and S100A14 are likely to be polymorphisms and match inter-individual variations that we have found in BCMP 84. Analysis of the protein sequence reveals no protein motifs that might indicate a particular function or cellular location for BCMP 84.

### Expression of BCMP 84 mRNA in human tissues

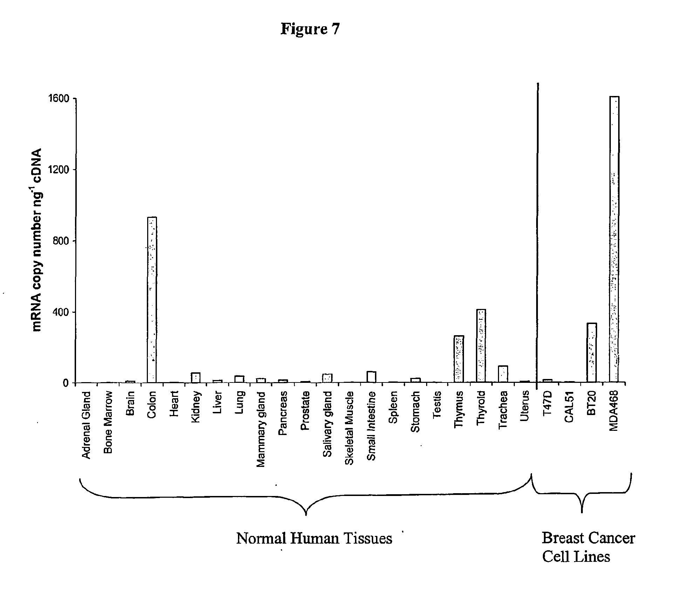

We used real time quantitative RT-PCR (Heid, C.A., Stevens, J., Livak, K.J. & Williams, P.M. Real time quantitative PCR. *Genome Res.* **6**, 986-994 (1996); Morrison, T.B., Weis, J.J. & Wittwer, C.T. Quantification of low-copy transcripts by continuous SYBR Green I monitoring during amplification. *Biotechniques* **24,** 954-958 (1998)) to analyse the distribution of BCMP 84 mRNA in normal human tissues and breast cancer cell lines (Fig 7).. The primers used for PCR were as follows: sense, 5' TCTGTGCACTCTGTCTTGGA 3', antisense, 5' TAGCCAGCTCCTCTCTGTT 3'. Reactions containing 10ng cDNA, prepared as described above, SYBR green sequence detection reagents (PE Biosystems) and sense and antisense primers were assayed on an ABI7700 sequence detection system (PE Biosystems). The PCR conditions were 1 cycle at 50°C for 2 min, 1 cycle at 95°C for 10 min, and 40 cycles of 95°C for 15s, 55°C for 1min. The accumulation of PCR product was measured in real time as the increase in SYBR green fluorescence, and the data were analysed using the Sequence Detector program vl .6.3 (PE Biosystems). Standard curves relating initial template copy number to fluorescence and amplification cycle were generated using the amplified=PCR product as a template, and were used to calculate BCMP 84 copy number in each sample.

The distribution of BCMP 84 mRNA was restricted to a few tissues, with the highest levels of expression in colon, thyroid and thymus (260-930 copies ng⁻¹ cDNA), and only low levels of BCMP 84 message detected in other normal tissues, including mammary gland. BCMP 84 mRNA was detected in BT-20 and MDA-MB-468 cells (300 and 1600 copies ng⁻¹ cDNA respectively), but not in T-47D or CAL51 breast carcinoma lines.

### Chromosomal localisation

### Radiation Hybrid mapping

Chromosomal localisation of the BCMP 84 gene was achieved by screening the Genebridge 4 Radiation Hybrid panel (Research Genetics Inc.) using the following pair of primers derived from the 3' untranslated region:
sense, 5' TCAGCTTCCTTCCCCAGGTC 3';
antisense, 5' CCCAGCTCCATTATTCA 3'.

The PCR conditions for amplification of BCMP 84 sequences were denaturation at 94°C for 30s, followed by annealing and extension at 55°C for 30s (40 cycles), using *Taq* DNA polymerase (Qiagen) and 25ng DNA per reaction. The primers amplified the expected 215bp fragment from the positive hybrid cell line DNAs and human genomic DNA, and failed to amplify product from hamster genomic DNA (control).

The radiation hybrid mapping data were submitted to the Whitehead Institute/MIT Centre for Genome research STS mapping server (http://carbon.wi.mit.edu:8000/cgi-bin/contig/rhmapper.pl) for analysis.

Radiation Hybrid mapping localised the BCMP 84 gene to chromosome 1q21 between the STS markers AFM291xh1 and AFM220xf8 within the S 100 calcium binding protein gene cluster. Thus, although BCMP 84 shows only limited homology to the other S100 family members and lacks obvious calcium binding domains, it is clearly related to this large family of proteins.

### EXAMPLE 5: TISSUE DISTRIBUTION ANALYSIS

### MATERIALS AND METHODS

### Quantitative mRNA analysis

BCMP 7 XAG (AF088867) and 23 NCAM2 (015394) are not intended to form part of the invention. The level of mRNA of BCMPs 7, 17 and 23, , *i.e.,* putative secreted protein XAG (AF088867), BST2 (Q10589) and NCAM2 (015394) in different cell types was determined by quantitative real-time PCR, using the double-stranded DNA dye, SYBR Green I, using methodologies and reagents as described in Morrison *et al, Biotechniques* (1998) **24:** 954-8, using the ABI7700 (PE Biosystems, UK) sequence detection system.

In summary, quantification of mRNA levels was achieved by generating a standard curve, using the purified (Qiagen, UK) PCR product as a template. PCRs containing 10⁴, 10⁵, 10⁶ and 10⁷ template molecules were performed in triplicate to determine the cycle number at which the amplification signal enters the log linear range (the threshold cycle, Ct). The standard curve thus defines the relationship between Ct and template concentration, and can be used to derive the initial template concentration in an unknown sample from its corresponding Ct value. Samples were run in duplicate, each using 10ng of first strand cDNA as the starting material.

The human tissue cDNAs were derived by oligo dT primed reverse transcription (SuperscriptII - Life Technologies, UK) from RNAs (Clontech, USA) pooled from multiple human sources. Total RNAs for human cell lines T47D, Cal51, MDA-MB468, PC3, PC3M, and BT20 were generated from tissue culture samples extracted with Trizol (Life Technologies, UK).

The primer sequences used to measure the levels of putative secreted protein XAG, BST2, and NCAM2 mRNAs were:

| XAG | |
|---|---|
| forward | agataccacagtcaaacctg |
| reverse | gcactcatccaagtgatgaa |

| BST2 | |
|---|---|
| forward | gatggagtgtcgcaatgtcacc |
| reverse | agacgcgtcctgaagcttatgg |

| NCAM2 | |
|---|---|
| forward | gatcatagagctgtcgcagacc |
| reverse | tgtagtctccttgtccctttcc |

### RESULTS

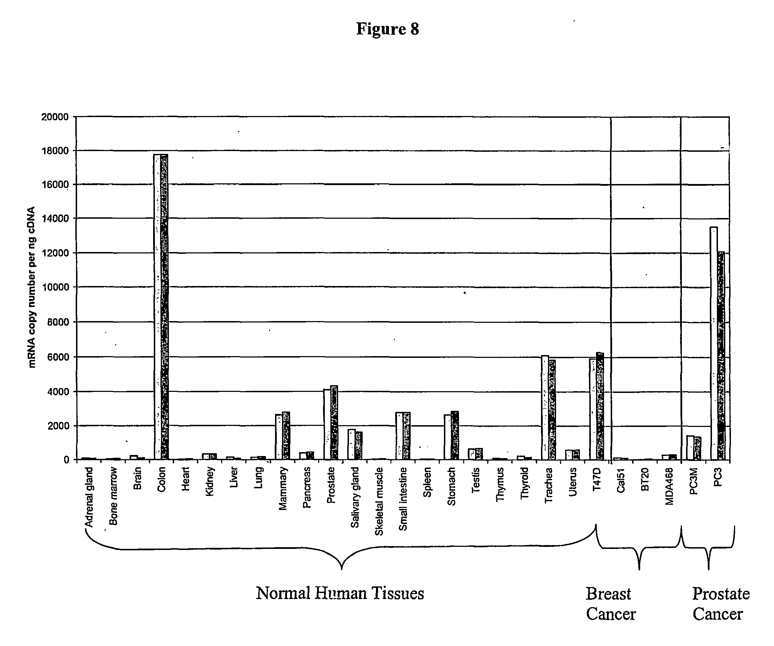

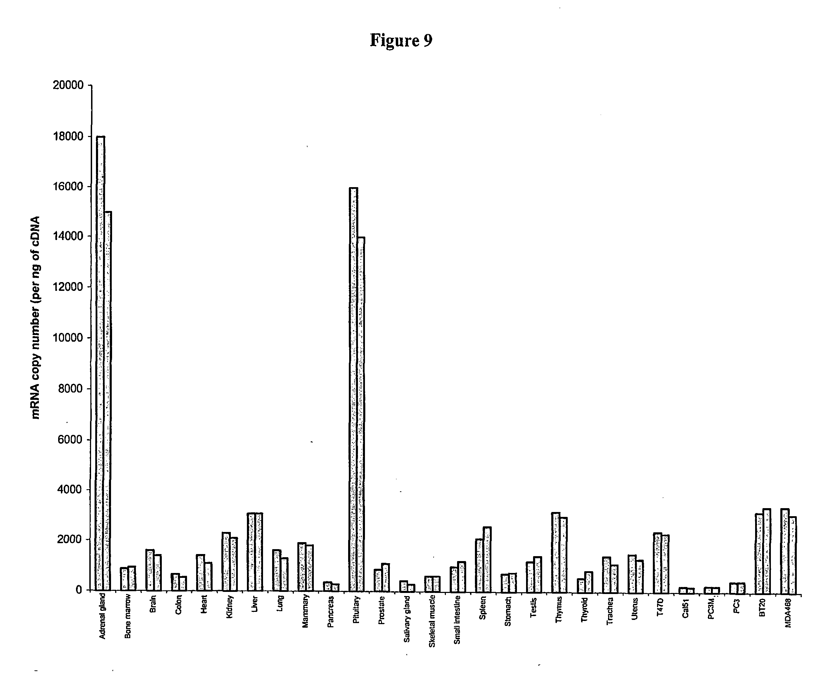

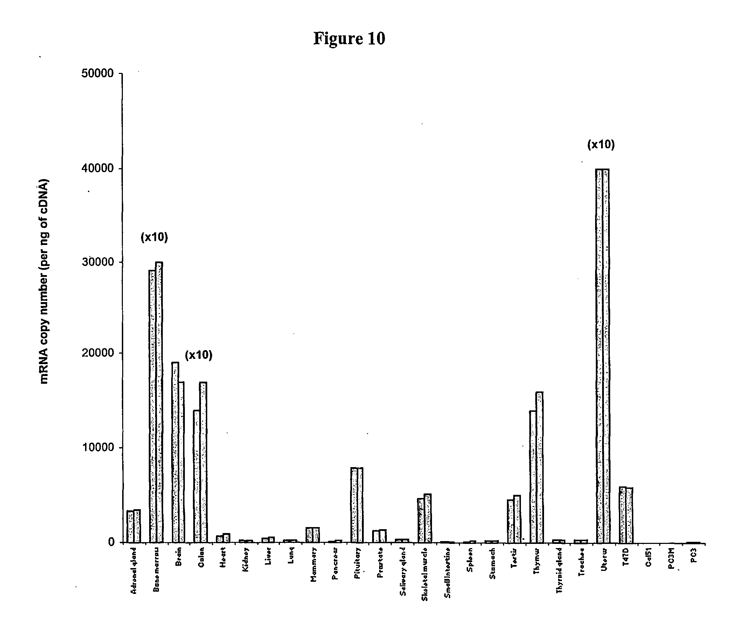

The results are shown in Figures 8-10. In the figures, the "x10" annotation means that the value for that sample is 10x higher than indicated.

## Claims

1. The use of an antibody capable of immunospecific binding to BST-2 (BCMP-17) or a fragment or derivative thereof which comprises the binding domain of the antibody, in the manufacture of a medicament for the treatment of breast cancer.

2. The use according to claim 1 wherein the antibody is monoclonal or humanised.

3. The use according to claim 1 or 2 wherein the antibody is conjugated to a therapeutic agent or drug moiety.

4. The use of BST-2 (BCMP-17) and/or one or more antigenic or immunogenic fragments or derivatives thereof, in the manufacture of a medicament for the treatment or prophylaxis of breast cancer.

5. The use according to claim 4 wherein the medicament is capable of eliciting an immune response in a subject.

6. The use according to claim 5 wherein the medicament is a vaccine.

7. A method for screening, diagnosis or prognosis of breast cancer in a subject, or for monitoring the effect of an anti-breast cancer drug or therapy administered to a subject, comprising quantitatively detecting BST-2 (BCMP-17) in a sample from the subject.

8. The method according to claim 7, wherein the sample is a sample of breast tissue.

9. The method according to claim 7 or 8, wherein the step of quantitatively detecting comprises:
a) contacting the sample with an antibody to capture BST-2 (BCMP-17); and
b) detecting the captured BST-2 using a directly or indirectly labelled detection reagent.

10. A method for screening, diagnosis or prognosis of breast cancer in a subject or for monitoring the effect of an anti-breast cancer drug or therapy administered to a subject, comprising:
a) contacting one or more oligonucleotide probes comprising 10 or more consecutive nucleotides complementary to a nucleotide sequence encoding BST-2 (BCMP-17), with an RNA obtained from a biological sample from the subject or with cDNA copied from the RNA, wherein said contacting occurs under conditions that permit hybridization of the probe to the nucleotide sequence if present;
b) detecting hybridization, if any, between the probe and the nucleotide sequence; and
c) comparing the hybridization, if any, detected in step b) with the hybridization detected in a control sample, or with a previously determined reference range.

11. A method of screening, diagnosis or prognosis of breast cancer in a subject or for monitoring the effect of an anti-breast cancer drug or therapy administered to a subject, which comprises:
a) bringing into contact with a sample to be tested BST-2 (BCMP-17), or one or more antigenic or immunogenic fragments or derivatives thereof; and
b) detecting the presence of antibodies to BST-2.

12. The *in vitro* use of BST-2 (BCMP 17) and/or one or more antigenic or immunogenic fragments thereof; or an antibody capable of immunospecific binding to BST-2, or a fragment or derivative thereof which comprises the binding domain of the antibody; for screening, diagnosis or prognosis of breast cancer in a subject or for monitoring the effect of an anti-breast cancer drug or therapy administered to a subject.

13. A method of screening for anti-breast cancer agents that interact with BST-2 (BCMP-17) or a biologically active portion thereof, the method comprising:
contacting BST-2, or a biologically active portion thereof, with a candidate agent; and
determining the ability of the candidate agent to interact with the BST-2, or a biologically active portion thereof.

14. A method of screening for anti-breast cancer agents that modulate the activity of BST-2 (BCMP-17) or a biologically active portion thereof, the method comprising:
in a first aliquot, contacting a candidate agent with BST-2, or a biologically active portion thereof; and
comparing the activity of BST-2 or a biologically active portion thereof in the first aliquot after addition of the candidate agent with the activity of BST-2 or a biologically active portion thereof in a control aliquot, or with a previously determined reference range.

15. A method of screening for anti-breast cancer agents that modulate the expression or activity of BST-2 (BCMP-17), comprising:
contacting an enzyme which is responsible for the production or degradation of BST-2 with a candidate agent;
detecting modulation of the activity of said enzyme.

16. A method of screening for anti-breast cancer agents that modulate the expression or activity of BST-2 (BCMP-17), comprising:
contacting a first group of cells expressing BST-2 with a candidate agent;
contacting a second group of cells expressing BST-2 with a control agent; and
comparing the level of expression or activity BST-2 or expression of mRNA encoding BST-2 in the first and second groups of cells, or comparing the level of induction of a cellular second messenger in the first and second groups of cells.

17. A method of screening for or identifying anti-breast cancer agents that modulate the expression or activity of BST-2 (BCMP-17), the method comprising:
administering a candidate agent to a first group of non-human mammals;
administering a control agent to a second group of non-human mammals; and
comparing the level of expression or activity of BST-2 or expression of mRNA encoding BST-2 in the first and second groups, or comparing the level of induction of a cellular second messenger in the first and second groups.

18. The method of claim 17, wherein the mammals are animal models for breast cancer.

19. The use of a BST-2 (BCMP-17) anti-sense nucleotide or ribozyme which interacts with, or modulates the activity of BST-2 (BCMP-17) in the manufacture of a medicament for the treatment of breast cancer.

## Patentansprüche

1. Verwendung von einem Antikörper, der zur immunospezifischen Bindung an BST-2 (BCMP-17) oder an ein Fragment oder Derivat davon fähig ist, welches die Bindungsdomäne des Antikörpers umfasst, für die Herstellung eines Medikaments für die Behandlung von Brustkrebs.

2. Verwendung nach Anspruch 1, wobei es sich bei dem Antikörper um einen monoklonalen oder humanisierten Antikörper handelt.

3. Verwendung nach Anspruch 1 oder 2, wobei der Antikörper zu einem therapeutischen Agens oder einem Medikamentenanteil konjugiert ist.

4. Verwendung von BST-2 (BCMP-17) und/oder einem oder mehreren antigenen oder immunogenen Fragmenten oder Derivat(en) davon, für die Herstellung eines Medikaments für die Behandlung oder Prophylaxe von Brustkrebs.

5. Verwendung nach Anspruch 4, wobei das Medikament dazu in der Lage ist, eine Immunantwort in einem Subjekt hervorzurufen.

6. Verwendung nach Anspruch 5, wobei das Medikament ein Impfstoff ist.

7. Verfahren für Screening, Diagnose oder Prognose von Brustkrebs in einem Subjekt, oder für das Überwachen der Wirkung eines Medikaments gegen Brustkrebs oder einer Therapie gegen Brustkrebs, welche(s) einem Subjekt verabreicht wird, welches den quantitativen Nachweis von BST-2 (BCMP-17) in einer Probe von dem Subjekt umfasst.

8. Verfahren nach Anspruch 7, wobei die Probe eine Probe aus dem Brustgewebe ist.

9. Verfahren nach Anspruch 7 oder 8, wobei der Schritt des quantitativen Nachweises umfasst:
a) In Kontakt bringen der Probe mit einem Antikörper, um BST-2 (BCMP-17) zu binden; und
b) Nachweisen des gebundenen BST-2 mithilfe eines direkt oder indirekt markierten Nachweisreagens.

10. Verfahren für Screening, Diagnose oder Prognose von Brustkrebs in einem Subjekt oder für das Überwachen der Wirkung eines Medikaments oder einer Therapie gegen Brustkrebs, welche(s) an ein Subjekt verabreicht wird, wobei das Verfahren umfasst:
a) In Kontakt bringen einer oder mehrerer Oligonukleotidesonden, welche 10 oder mehr konsekutive Nukleotide umfassen, die komplementär zu einer Nukleotidsequenz sind, die für BST-2 (BCMP-17) kodiert, mit einer RNA, die aus einer biologischen Probe aus dem Subjekt gewonnen wurde, oder mit cDNA, die von der RNA kopiert wurde, wobei das in Kontakt bringen unter Bedingungen stattfindet, welche die Hybridisierung der Sonde zu der Nukleotidsequenz, falls diese vorliegt, ermöglichen;
b) Nachweisen von Hybridisierung, falls stattfindend, zwischen der Sonde und der Nukleotidsequenz; und
c) Vergleichen der Hybridisierung, falls stattfindend, wie nachgewiesen in Schritt b), wobei die Hybridisierung in einer Kontrollprobe nachgewiesen wird oder mithilfe eines zuvor festgelegten Referenzbereichs.

11. Verfahren für Screening, Diagnose oder Prognose von Brustkrebs in einem Subjekt oder für das Überwachen der Wirkung eines Medikaments oder einer Therapie gegen Brustkrebs, welche(s) einem Subjekt verabreicht wird, wobei das Verfahren umfasst:
a) In Kontakt bringen mit einer Probe, die auf BST-2 (BCMP-17) getestet werden soll, oder einem oder mehreren antigenen oder immunogenen Fragmenten oder Derivaten davon, und
b) Nachweisen des Vorhandenseins von Antikörpern gegen BST-2.

12. *In-vitro*-Verwendung von BST-2 (BCMP 17) und/oder einem oder mehreren antigenen oder immunogenen Fragmenten davon, oder einem Antikörper, der zu einer immunospezifischen Bindung an BST-2, oder an ein Fragment oder Derivat davon fähig ist, welches die Bindungsdomäne des Antikörpers umfasst, für Screening, Diagnose oder Prognose von Brustkrebs in einem Subjekt oder für das Überwachen der Wirkung eines Medikaments oder einer Therapie gegen Brustkrebs, welche(s) einem Subjekt verabreicht wird.

13. Verfahren für Screening von Agenzien gegen Brustkrebs, die mit BST-2 (BCMP-17) oder einem biologisch aktiven Anteil davon interagieren, wobei das Verfahren umfasst:
In Kontakt bringen von BST-2 oder einem biologisch aktiven Teil davon mit einem möglichen Agens, und
Bestimmen der Fähigkeit des möglichen Agens, mit dem BST-2 oder einem biologisch aktiven Anteil davon zu interagieren.

14. Verfahren für Screening für Agenzien gegen Brustkrebs, die die Aktivität von BST-2 (BCMP-17) oder einem biologisch aktiven Anteil davon verändern, wobei das Verfahren umfasst:
in einem ersten Aliquot, in Kontakt bringen eines möglichen Agens mit BST-2 oder einem biologisch aktiven Anteil davon; und
Vergleichen der Aktivität von BST-2 oder einem biologisch aktiven Anteil davon in dem ersten Aliquot nach Zugabe des möglichen Agens mit der Aktivität von BST-2 oder einem biologisch aktiven Anteil davon in einem Kontrollaliquot oder mithilfe eines zuvor festgelegten Referenzbereichs.

15. Verfahren für Screening von Agenzien gegen Brustkrebs, die die Expression oder die Aktivität von BST-2 (BCMP-17) verändern, welches umfasst:
In Kontakt bringen mit einem Enzym, welches verantwortlich ist für die Produktion oder den Abbau von BST-2 mit einem möglichen Agens;
Nachweisen von Veränderung der Aktivität des Enzyms.

16. Verfahren für Screening von Agenzien gegen Brustkrebs, die die Expression oder die Aktivität von BST-2 (BCMP-17) verändern, welches umfasst:
In Kontakt bringen mit einer ersten Gruppe von Zellen, die BST-2 mit einem möglichen Agens exprimieren;
In Kontakt bringen mit einer zweiten Gruppe von Zellen, die BST-2 mit einem Kontrollagens exprimieren, und
Vergleichen des Ausmaßes an Expression oder Aktivität von BST-2 oder Expression von mRNA, die für BST-2 in der ersten und zweiten Gruppe von Zellen kodiert, oder Vergleichen des Ausmaßes an Induktion eines zellulären zweiten Messengers in der ersten und zweiten Gruppe von Zellen.

17. Verfahren für Screening oder Identifizierung von Agenzien gegen Brustkrebs, die die Expression oder die Aktivität von BST-2 (BCMP-17) verändern, wobei das Verfahren umfasst:
Verabreichen eines möglichen Agens an eine erste Gruppe von nicht menschlichen Säugetieren;
Verabreichen eines Kontrollagens an eine zweite Gruppe von nicht menschlichen Säugetieren, und
Vergleichen des Ausmaßes von Expression oder Aktivität von BST-2 oder Expression von mRNA, die für BST-2 in der ersten und zweiten Gruppe kodiert, oder Vergleichen des Ausmaßes an Induktion eines zellulären zweiten Messengers in der ersten und zweiten Gruppe.

18. Verfahren nach Anspruch 17, wobei die Säugetiere Tiermodelle für Brustkrebs sind.

19. Verwenden von einem BST-2 (BCMP-17)-Antisense-Nukleotid oder -ribozym, welches mit BST-2 (BCMP-17) bei der Herstellung eines Medikaments für die Behandlung von Brustkrebs interagiert oder dessen Aktivität verändert.

## Revendications

1. Utilisation d'un anticorps susceptible de se lier de manière immunospécifique à BST-2 (BCMP-17), ou d'un fragment ou dérivé de celui-ci qui comprend le domaine de liaison de l'anticorps, dans la fabrication d'un médicament destiné au traitement du cancer du sein.

2. Utilisation selon la revendication 1, **caractérisée en ce que** l'anticorps est monoclonal ou humanisé.

3. Utilisation selon la revendication 1 ou 2, **caractérisée en ce que** l'anticorps est conjugué à un fragment d'agent thérapeutique ou de médicament.

4. Utilisation de BST-2 (BCMP-17) et/ou d'un ou de plusieurs fragments ou dérivés antigéniques ou immunogènes de celui-ci, dans la fabrication d'un médicament destiné au traitement ou à la prophylaxie du cancer du sein.

5. Utilisation selon la revendication 4, **caractérisée en ce que** le médicament est susceptible de provoquer une réponse immunitaire chez un sujet.

6. Utilisation selon la revendication 5, **caractérisée en ce que** le médicament est un vaccin.

7. Procédé de dépistage, de diagnostic ou de pronostic du cancer du sein chez un sujet, ou de suivi de l'effet d'un médicament ou d'une thérapie anti-cancer du sein administré à un sujet, comprenant la détection quantitative de BST-2 (BCMP-17) dans un échantillon provenant du sujet.

8. Procédé selon la revendication 7, **caractérisé en ce que** l'échantillon est un échantillon de tissu mammaire.

9. Procédé selon la revendication 7 ou 8, **caractérisé en ce que** l'étape de détection quantitative comprend :
a) la mise en contact de l'échantillon avec un anticorps pour capturer BST-2 (BCMP-17) ; et
b) la détection du BST-2 capturé en utilisant un réactif de détection marqué directement ou indirectement.

10. Procédé de dépistage, de diagnostic ou de pronostic du cancer du sein chez un sujet, ou de suivi de l'effet d'un médicament ou d'une thérapie anti-cancer du sein administré à un sujet, comprenant :
a) la mise en contact d'une ou de plusieurs sondes oligonucléotidiques comprenant 10 nucléotides consécutifs, ou plus, complémentaires d'une séquence nucléotidique codant pour BST-2 (BCMP-17), avec un ARN obtenu à partir d'un échantillon biologique du sujet ou avec un ADNc copié à partir de l'ARN, ladite mise en contact étant réalisée dans des conditions permettant l'hybridation de la sonde à la séquence nucléotidique si elle est présente ;
b) la détection de l'hybridation, le cas échéant, entre la sonde et la séquence nucléotidique ; et
c) la comparaison de l'hybridation, le cas échéant, détectée à l'étape b) avec l'hybridation détectée dans un échantillon témoin, ou avec une gamme de référence préalablement déterminée.

11. Procédé de dépistage, de diagnostic ou de pronostic du cancer du sein chez un sujet ou de suivi de l'effet d'un médicament ou d'une thérapie anti-cancer du sein administré à un sujet, qui comprend :
a) la mise en contact avec un échantillon à tester de BST-2 (BCMP-17), ou d'un ou de plusieurs fragments ou dérivés antigéniques ou immunogènes de celui-ci ; et
b) la détection de la présence d'anticorps anti-BST-2.

12. Utilisation *in vitro* de BST-2 (BCMP-17) et/ou d'un ou de plusieurs fragments antigéniques ou immunogènes de celui-ci ; ou d'un anticorps susceptible de se lier de manière immunospécifique à BST-2, ou d'un fragment ou dérivé de celui-ci qui comprend le domaine de liaison de l'anticorps ; pour le dépistage, le diagnostic ou le pronostic du cancer du sein chez un sujet ou pour le suivi de l'effet d'un médicament ou d'une thérapie anti-cancer du sein administré à un sujet.

13. Procédé de criblage d'agents anti-cancer du sein qui interagissent avec BST-2 (BCMP-17) ou une portion biologiquement active de celui-ci, le procédé comprenant:
la mise en contact de BST-2, ou d'une portion biologiquement active de celui-ci, avec un agent candidat ; et
la détermination de l'aptitude de l'agent candidat à interagir avec BST-2, ou une portion biologiquement active de celui-ci.

14. Procédé de criblage d'agents anti-cancer du sein qui modulent l'activité de BST-2 (BCMP-17) ou d'une portion biologiquement active de celui-ci, le procédé comprenant :
dans une première aliquote, la mise en contact d'un agent candidat avec BST-2, ou une portion biologiquement active de celui-ci ; et
la comparaison de l'activité de BST-2 ou d'une portion biologiquement active de celui-ci dans la première aliquote après l'ajout de l'agent candidat, avec l'activité de BST-2 ou d'une portion biologiquement active de celui-ci dans une aliquote témoin, ou avec une gamme de référence préalablement déterminée.

15. Procédé de criblage d'agents anti-cancer du sein qui modulent l'expression ou l'activité de BST-2 (BCMP-17), comprenant:
la mise en contact d'une enzyme responsable de la production ou la dégradation de BST-2 avec un agent candidat ;
la détection de la modulation de l'activité de ladite enzyme.

16. Procédé de criblage d'agents anti-cancer du sein qui modulent l'expression ou l'activité de BST-2 (BCMP-17), comprenant :
la mise en contact d'un premier groupe de cellules exprimant BST-2 avec un agent candidat ;
la mise en contact d'un deuxième groupe de cellules exprimant BST-2 avec un agent témoin ; et
la comparaison du niveau d'expression ou d'activité de BST-2 ou d'expression d'ARNm codant pour BST-2 dans les premier et deuxième groupes de cellules, ou la comparaison du niveau d'induction d'un second messager cellulaire dans les premier et deuxième groupes de cellules.

17. Procédé de criblage ou d'identification d'agents anti-cancer du sein qui modulent l'expression ou l'activité de BST-2 (BCMP-17), le procédé comprenant:
l'administration d'un agent candidat à un premier groupe de mammifères non humains ;
l'administration d'un agent témoin à un deuxième groupe de mammifères non humains ; et
la comparaison du niveau d'expression ou d'activité de BST-2 ou d'expression d'ARNm codant pour BST-2 dans les premier et deuxième groupes, ou la comparaison du niveau d'induction d'un second messager cellulaire dans les premier et deuxième groupes.

18. Procédé selon la revendication 17, **caractérisé en ce que** les mammifères sont des modèles animaux pour le cancer du sein.

19. Utilisation d'un ribozyme ou d'un nucléotide anti-sens de BST-2 (BCMP-17) qui interagit avec, ou module l'activité de BST-2 (BCMP-17), dans la fabrication d'un médicament destiné au traitement du cancer du sein.
